(19) Europäisches Patentamt
European Patent Office
Office européen des brevets

(11) EP 2 900 221 B1

(12) **EUROPEAN PATENT SPECIFICATION**

(45) Date of publication and mention of the grant of the patent:
**06.03.2019 Bulletin 2019/10**

(21) Application number: **13848127.0**

(22) Date of filing: **27.09.2013**

(51) Int Cl.:
*A61K 9/16* (2006.01)
*A61K 9/48* (2006.01)
*A61K 9/20* (2006.01)
*A61K 9/14* (2006.01)
*A61K 31/5517* (2006.01)

(86) International application number:
**PCT/IB2013/003026**

(87) International publication number:
**WO 2014/068402 (08.05.2014 Gazette 2014/19)**

(54) **PHARMACEUTICAL FORMULATION CONTAINING THIENOTRIAZOLODIAZEPINE COMPOUNDS**

PHARMAZEUTISCHE FORMULIERUNG MIT THIENOTRIAZOLDIAZEPINVERBINDUNGEN

FORMULATION PHARMACEUTIQUE CONTENANT DES COMPOSÉS THIENOTRIAZOLODIAZEPINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.09.2012 US 201261707465 P**
**14.03.2013 US 201361782882 P**
**06.06.2013 US 201361831811 P**

(43) Date of publication of application:
**05.08.2015 Bulletin 2015/32**

(73) Proprietor: **Oncoethix GmbH**
**6006 Luzern (CH)**

(72) Inventors:
- **GAUTSCHI, Jeff**
**Bend, OR 97701 (US)**
- **MINIKIS, Ryan**
**Bend, OR 97702 (US)**

(74) Representative: **Jaap, David Robert**
**Merck Sharp & Dohme Corp.**
**European Patent Department**
**Hertford Road**
**Hoddesdon, Hertfordshire EN11 9BU (GB)**

(56) References cited:
**EP-A1- 1 297 836**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 900 221 B1

## Description

FIELD OF INVENTION

The present disclosure describes solid dispersions of a thienotriazolodiazepine compound which has improved solubility and bioavailability.

BACKGROUND OF THE INVENTION

The thienotriazolodiazepine compound, described herein below, has been shown to inhibit the binding of acetylated histone H4 to the tandem bromodomain (BRD)-containing family of transcriptional regulators known as the BET (bromodomains and extraterminal) proteins, which include BRD2, BRD3, and BRD4. *See* U.S. Patent Application Publication No. 2010/0286127 A1. The BET proteins have emerged as major epigenetic regulators of proliferation and differentiation and also have been associated with predisposition to dyslipidemia or improper regulation of adipogenesis, elevated inflammatory profile and risk for cardiovascular disease and type 2 diabetes, and increased susceptibility to autoimmine diseases such as rheumatoid arthritis and systemic lupus erythematosus as reported by Denis, G.V. "Bromodomain coactivators in cancer, obesity, type 2 diabetes, and inflammation," Discov Med 2010; 10:489-499. Accordingly, the thienotriazolodiazepine compound described herein may be useful for treatment of various cancers, cardiovascular disease, type 2 diabetes, and autoimmune disorders such as rheumatoid arthritis and systemic lupus erythematosus.

The thienotriazolodiazepine compound described herein below, present highly specific difficulties in relation to administration generally and the preparation of galenic compositions in particular, including the particular problems of drug bioavailability and variability in inter- and intra-patient dose response, necessitating development of a non-conventional dosage form with respect to the practically water-insoluble properties of the thienotriazolodiazepine.

Compositions containing thienotriazolodiazepine for controlling release pH range and/or speed, and their method of production have been reported in European Patent Application Publication No. EP 1297836 A1.

Previously, it had been found that the thienotriazolodiazepine compound described herein could be formulated with the carrier ethyl acrylate-methyl methacrylate-trimethylammonioethyl methacrylate chloride copolymer (Eudragit RS, manufactured by Rohm) to provide an oral formulation that preferentially released the pharmaceutical ingredient in the lower intestine for treatment of inflammatory bowel diseases such as ulcerative colitis and Crohn's disease as reported in U.S. Patent Application Publication No. 20090012064 A1. Through various experiments including animal tests, it was found that that for inflammatory bowel diseases, the thienotriazolodiazepine compound described herein releases in a lesion and a direct action thereof on the inflammatory lesion were more important than the absorption of the thienotriazolodiazepine compound into circulation from the gastrointestinal tract. However, for many other disease conditions high absorption of the thienotriazolodiazepine compound described herein into the circulation from gastrointestinal tract is required. Accordingly, a need exists for formulations of the thienotriazolodiazepine compound described herein that can provide high absorption of the thienotriazolodiazepine compound into the circulation from gastrointestinal tract.

BRIEF SUMMARY OF THE INVENTION

In one embodiment, the present disclosure provides for a solid dispersion comprising an amorphous thienotriazolodiazepine compound (S)-2-[4-(4-chlorophenyl)-2,3,9-trimethyl-6H-thieno[3,2-f] [1,2,-4]triazolo[4,3-a][1,4]diazepin-6-yl]-N-(4-hydroxyphenyl)acetamide, a pharmaceutically acceptable salt thereof or a hydrate thereof; and a pharmaceutically acceptable polymer, wherein the pharmaceutically acceptable polymer is hydroxypropylmethylcellulose acetate succinate and wherein the solid dispersion has a thienotriazolodiazepine compound to hydroxypropylmethylcellulose acetate succinate (HPMCAS), weight ratio of 1:3 to 1:1.

In some such embodiments, the solid dispersion exhibits a single glass transition temperature (Tg) inflection point ranging from about 130 °C to about 140 °C. In some such embodiments, a concentration of the thienotriazolodiazepine compound after exposure to the relative humidity of 75 % at 40 °C for at least one month is at least 90 % of the concentration the amorphous thienotriazolodiazepine compound prior to such exposure.

In another embodiment, the solid dispersion is obtained by spray drying.

In another embodiment, the solid dispersion exhibits an X-ray powder diffraction pattern substantially free of diffraction lines associated with the crystalline thienotriazolodiazepine compound described herein.

In yet another embodiment, the solid dispersion provides an area under the curve (AUC) value that is at least 0.5 times that of a corresponding AUC value provided by a control composition administered intravenously, wherein the control composition comprises an equivalent quantity of the crystalline thienotriazolodiazepine compound described herein.

In still yet another embodiment, the solid dispersion provides a concentration, of the amorphous thienotriazolodiazepine compound, in an aqueous in vitro test medium at pH between 5.0 to 7.0, of at least 5-fold greater than a concentration of a crystalline thienotriazolodiazepine compound described herein without polymer, in a control in vitro test medium at pH between 5.0 to 7.0 test medium.

In yet another embodiment, a concentration of the amorphous thienotriazolodiazepine compound, from the solid dispersion, in an aqueous in vitro test medium having a pH of 1.0 to 2.0, is at least 50% higher than a concentration of a crystalline thienotriazolodiazepine compound described herein without polymer in an in vitro test medium having a pH between 5.0 and 7.0.

In one embodiment, the concentration of the amorphous thienotriazolodiazepine compound, is at least 50% higher compared to a concentration of the thienotriazolodiazepine compound described herein, from a solid dispersion of the thienotriazolodiazepine compound described herein and a pharmaceutically acceptable polymer selected from the group consisting of: hypromellose phthalate and ethyl acrylate-methyl methacrylate-trimethylammonioethyl methacrylate chloride copolymer, wherein each solid dispersion was placed in an aqueous in vitro test medium having a pH of 1.0 to 2.0.

The present disclosure further provides for a pharmaceutical formulation comprising a spray dried solid dispersion, as described herein, and one or more pharmaceutically acceptable excipients selected from the group consisting of: lactose monohydrate; microcrystalline cellulose; croscarmellose sodium; colloidal silicon dioxide; magnesium stearate; and combinations thereof. In some embodiments, the pharmaceutical formulation has a bulk density ranging from 0.55 g/cc to 0.60 g/cc. In some embodiments, the pharmaceutical formation may be a pharmaceutical capsule. In some embodiments, the pharmaceutical formation may be a pharmaceutical tablet.

The present disclosure further provides for a pharmaceutical formulation comprising 10-15 wt. % of a spray dried solid dispersion, as described herein, and hydroxypropylmethylcellulose acetate succinate (HPMCAS), wherein the thienotriazolodiazepine compound is amorphous in the dispersion and has a thienotriazolodiazepine compound to hydroxypropylmethylcellulose acetate succinate (HPMCAS), weight ratio of 1:3 to 1:1;45 -50 wt. % of lactose monohydrate; 35-40 wt. % of microcrystalline cellulose; 4-6 wt. % of croscarmellose sodium; 0.8-1.5 wt. % of colloidal silicon dioxide; and 0.8-1.5 wt. % of magnesium stearate.

BRIEF DESCRIPTION OF THE DRAWINGS

The foregoing summary, as well as the following detailed description of embodiments of the pharmaceutical compositions including thienotriazolodiazepine formulations and methods of the present invention, will be better understood when read in conjunction with the appended drawings of exemplary embodiments.

In the drawings:

Figure 1A illustrates dissolution profile of a comparator formulation comprising a solid dispersion comprising 25% compound (1-1) and Eudragit L100-55;
Figure 1B illustrates dissolution profile of a comparator formulation comprising a solid dispersion comprising 50% compound (1-1) and Eudragit L100-55;
Figure 1C illustrates dissolution profile of an illustrative formulation comprising a solid dispersion comprising 25% compound (1-1) and polyvinylpyrrolidone (PVP);
Figure 1D illustrates dissolution profile of an illustrative formulation comprising a solid dispersion comprising 50% compound (1-1) and PVP;
Figure IE illustrates dissolution profile of an illustrative formulation comprising a solid dispersion comprising 25% compound (1-1) and PVP-vinyl acetate (PVP-VA);
Figure IF illustrates dissolution profile of an illustrative formulation comprising a solid dispersion comprising 50% compound (1-1) and PVP-VA;
Figure 1G illustrates dissolution profile of an exemplary formulation comprising a solid dispersion comprising 25% compound (1-1) and hypromellose acetate succinate (HPMCAS-M);
Figure 1H illustrates dissolution profile of an exemplary formulation comprising a solid dispersion comprising 50% compound (1-1) and HPMCAS-M;
Figure 1I illustrates dissolution profile of an illustrative formulation comprising a solid dispersion comprising 25% compound (1-1) and hypromellose phthalate (HPMCP-HP55);
Figure 1J illustrates dissolution profile of an illustrative formulation comprising a solid dispersion comprising 50% compound (1-1) and HMCP-HP55;
Figure 2A illustrates results of *in vivo* screening of an illustrative formulation comprising a solid dispersion of 25% compound (1-1) and PVP;
Figure 2B illustrates results of an *in vivo* screening of an exemplary formulation comprising a solid dispersion of 25% compound (1-1) and HPMCAS-M;
Figure 2C illustrates results of an *in vivo* screening of an exemplary formulation comprising a solid dispersion of

50% compound (1-1) and HPMCAS-M;
Figure 3 illustrates powder X-ray diffraction profiles of solid dispersions of compound (1-1);
Figure 4A illustrates modified differential scanning calorimetry trace for a solid dispersion of 25% compound (1-1) and PVP equilibrated under ambient conditions;
Figure 4B illustrates modified differential scanning calorimetry trace for a solid dispersion of 25% compound (1-1) and HPMCAS-M equilibrated under ambient conditions;
Figure 4C illustrates modified differential scanning calorimetry trace for a solid dispersion of 50% compound (1-1) and HPMCAS-M equilibrated under ambient conditions;
Figure 5 illustrates plot of glass transition temperature (Tg) versus relative hunidity (RH) for solid dispersions of 25% compound (1-1) and PVP or HMPCAS-M and 50% compound (1-1) and HPMCAS-MG;
Figure 6 illustrates modified differential scanning calorimetry trace for a solid dispersion of 25% compound (1-1) and PVP equilibrated under 75% relative humidity;
Figure 7 illustrates plasma concentration versus time curves for Compound (1-1) after 1 mg/kg intravenous dosing (solid rectangles) and 3 mg/kg oral dosing as 25% Compound (1-1):PVP (open circles), 25% Compound (1-1):HPMCAS-MG (open triangles), and 50% Compound (1-1):HPMCAS-MG (open inverted triangles). The inset depicts the same data plotted on a semilogarithmic scale;
Figure 8 illustrates plasma concentration versus time curves for Compound (1-1) after 3 mg/kg oral dosing as 25% Compound (1-1):PVP (open circles), 25% Compound (1-1):HPMCAS-MG (open triangles), and 50% Compound (1-1):HPMCAS-MG (open inverted triangles). The inset depicts the same data plotted on a semi-logarithmic scale;
Figure 9 illustrates a powder X-ray diffraction profile of solid dispersions of compound (1-1) in HPMCAS-MG at time zero of a stability test;
Figure 10 illustrates a powder X-ray diffraction profile of solid dispersions of compound (1-1) in HPMCAS-MG after 1 month at 40 °C and 75 % relative humidity;
Figure 11 illustrates a powder X-ray diffraction profile of solid dispersions of compound (1-1) in HPMCAS-MG after 2 months at 40 °C and 75 % relative humidity; and
Figure 12 illustrates a powder X-ray diffraction profile of solid dispersions of compound (1-1) in HPMCAS-MG after 3 month at 40 °C and 75 % relative humidity.

DETAILED DESCRIPTION OF THE INVENTION

The present subject matter will now be described more fully hereinafter with reference to the accompanying Figures and Examples, in which representative embodiments are shown. Unless otherwise defined, all technical and scientific terms used herein have the same meaning as commonly understood by one of ordinary skill in the art to which the subject matter pertains.

**I. Definitions:**

The term "alkyl group" as used herein refers to a saturated straight or branched hydrocarbon.
The term "substituted alkyl group" refers to an alkyl moiety having one or more substituents replacing a hydrogen or one or more carbons of the hydrocarbon backbone.
The term "alkenyl group" whether used alone or as part of a substituent group, for example, "$C_{1-4}$alkenyl(aryl)," refers to a partially unsaturated branched or straight chain monovalent hydrocarbon radical having at least one carbon-carbon double bond, whereby the double bond is derived by the removal of one hydrogen atom from each of two adjacent carbon atoms of a parent alkyl molecule and the radical is derived by the removal of one hydrogen atom from a single carbon atom. Atoms may be oriented about the double bond in either the cis (Z) or trans (E) conformation. Typical alkenyl radicals include ethenyl, propenyl, allyl(2-propenyl), butenyl and the like. Examples include $C_{2-8}$alkenyl or $C_{2-4}$alkenyl groups.
The term "$C_{(j-k)}$" (where *j* and *k* are integers referring to a designated number of carbon atoms) refers to an alkyl, alkenyl, alkynyl, alkoxy or cycloalkyl radical or to the alkyl portion of a radical in which alkyl appears as the prefix root containing from j to k carbon atoms inclusive. For example, $C_{(1-4)}$ denotes a radical containing 1, 2, 3 or 4 carbon atoms.
The terms "halo" or "halogen" as used herein refer to F, Cl, Br, or I.
The term "pharmaceutically acceptable salts" is art-recognized and refers to the relatively non-toxic, inorganic and organic acid addition salts, or inorganic or organic base addition salts of compounds, including, for example, those contained in compositions of the present invention.
The term "solid dispersion" as used herein refers to a group of solid products consisting of at least two different components, generally a hydrophilic carrier and a hydrophobic drug (active ingredient).
The term "chiral" is art-recognized and refers to molecules That have the property of non-superimposability of

the mirror image partner, while the term "achiral" refers to molecules which are superimposable on their mirror image partner. A "prochiral molecule" is a molecule that has the potential to be converted to a chiral molecule in a particular process.

The symbol "------" is used to denote a bond that may be a single, a double or a triple bond.

The term "enantiomer" as it used herein, and structural formulas depicting an enantiomer are meant to include the "pure" enantiomer free from its optical isomer as well as mixtures of the enantiomer and its optical isomer in which the enantiomer is present in an enantiomeric excess, e.g., at least 10%, 25%, 50%, 75%, 90%, 95%, 98%, or 99% enantiomeric excess.

The term "stereoisomers" when used herein consist of all geometric isomers, enantiomers or diastereomers.

The term "stereoselective synthesis" as it is used herein denotes a chemical or enzymatic reaction in which a single reactant forms an unequal mixture of stereoisomers during the creation of a new stereocenter or during the transformation of a pre-existing one, and are well known in the art. Stereoselective syntheses encompass both enantioselective and diastereoselective transformations. For examples, see Carreira, E. M. and Kvaerno, L., Classics in Stereoselective Synthesis, Wiley-VCH: Weinheim, 2009.

The term "spray drying" refers to processes which involve the atomization of the feed suspension or solution into small droplets and rapidly removing solvent from the mixture in a processor chamber where there is a strong driving force for the evaporation (i.e., hot dry gas or partial vacuum or combinations thereof).

The term "therapeutically effective amount" as used herein refers to any amount of a thienotriazolodiazepine of the present invention or any other pharmaceutically active agent which, as compared to a corresponding a patient who has not received such an amount of the thienotriazolodiazepine or the other pharmaceutically active agent, results in improved treatment, healing, prevention, or amelioration of a disease, disorder, or side effect, or a decrease in the rate of advancement of a disease or disorder.

The term "about" means +/ 10%.

Throughout this application and in the claims that follow, unless the context requires otherwise, the word "comprise", or variations such as "comprises" or "comprising", should be understood to imply the inclusion of a stated integer step or group of integers or steps but not the exclusion of any other integer or step or group of integers or steps.

It has now been found that the thienotriazolodiazepine compound, described herein below, can be formulated as a solid dispersion with pharmaceutically acceptable polymers, to provide an oral formulation that provides high absorption of the pharmaceutical ingredient into the circulation from the gastrointestinal tract. The pharmaceutically acceptable polymer is hypromellose acetate succinate (also called hydroxypropylmethylcellulose acetate succinate or HPMCAS).

In some embodiments, the hydroxypropylmethyl cellulose acetate succinates (HPMCAS), may include M grade having 9% acetyl/11% succinoyl (e.g., HPMCAS having a mean particle size of 5 μm (i.e., HPMCAS-MF, fine powder grade) or having a mean particle size of 1 mm (i.e., HPMCAS-MG, granular grade)), H grade having 12% acetyl/6% succinoyl (e.g., HPMCAS having a mean particle size of 5 μm (i.e., HPMCAS-HF, fine powder grade) or having a mean particle size of 1 mm (i.e., HPMCAS-HG, granular grade)), and L grade having 8% acetyl/15% succinoyl (e.g., HPMCAS having a mean particle size of 5 μm (i.e., HPMCAS-LF, fine powder grade) or having a mean particle size of 1 mm (i.e., HPMCAS-LG, granular grade).

## II. The Thienotriazolodiazepine Compound:

In one embodiment, the thienotriazolodiazepine compound, used in the formulations of the present invention is (S)-2-[4-(4-chlorophenyl)-2,3,9-trimethyl-6H-thieno[3,2-f][1,2,-4]triazolo[4,3-a][1,4]diazepin-6-yl]-N-(4-hydroxyphenyl)acetamide, as shown in Formula (1-1) below.

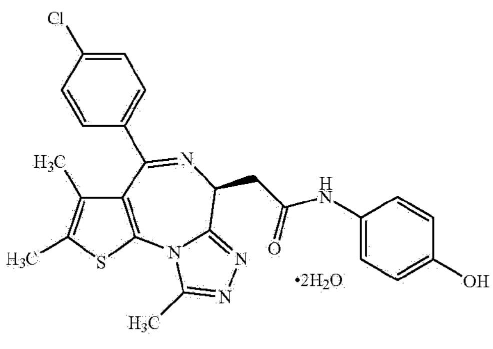

(1-1)

In some embodiments, the present invention provides pharmaceutically acceptable salts, hydrates, and isotopically-labeled forms of the thienotriazolodiazepine compound described herein. In one embodiment, pharmaceutically acceptable salts of the thienotriazolodiazepine compound include acid addition salts formed with inorganic acids. In one embodiment, pharmaceutically acceptable inorganic acid addition salts of the thienotriazolodiazepine include salts of hydrochloric, hydrobromic, hydroiodic, phosphoric, metaphosphoric, nitric and sulfuric acids. In one embodiment, pharmaceutically acceptable salts of the thienotriazolodiazepine compound includes acid addition salts formed with organic acids. In one embodiment, pharmaceutically acceptable organic acid addition salts of the thienotriazolodiazepine include salts of tartaric, acetic, trifluoroacetic, citric, malic, lactic, fumaric, benzoic, formic, propionic, glycolic, gluconic, maleic, succinic, camphorsulfuric, isothionic, mucic, gentisic, isonicotinic, saccharic, glucuronic, furoic, glutamic, ascorbic, anthranilic, salicylic, phenylacetic, mandelic, embonic (pamoic), methanesulfonic, ethanesulfonic, pantothenic, stearic, sulfinilic, alginic, galacturonic and arylsulfonic, for example benzenesulfonic and 4-methyl benzenesulfonic acids.

The present invention provides pharmaceutically acceptable isotopically-labeled forms of the thienotriazolodiazepine compound, described herein, wherein one or more atoms are replaced by atoms having the same atomic number, but an atomic mass or mass number different from the atomic mass or mass number usually found in nature. Examples of isotopes suitable for inclusion in the thienotriazolodiazepine compound include isotopes of hydrogen, e.g., $^{2}H$ and $^{3}H$, carbon, e.g., $^{11}C$, $^{13}C$ and $^{14}C$, chlorine, e.g., $^{36}Cl$, fluorine, e.g., $^{18}F$, iodine, e.g., $^{123}I$ and $^{125}I$, nitrogen, e.g., $^{13}N$ and $^{15}N$, oxygen, e.g., $^{15}O$, $^{17}O$ and $^{18}O$, and sulfur, e.g., $^{35}S$. Isotopically-labeled forms of the thienotriazolodiazepine compound generally can be prepared by conventional techniques known to those skilled in the art.

Certain isotopically-labeled forms of the compound described herein, for example those incorporating a radioactive isotope, are useful in drug and/or substrate tissue distribution studies. The radioactive isotopes tritium ($^{3}H$) and carbon-14 ($^{14}C$) are particularly useful for this purpose in view of their ease of incorporation and ready means of detection. Substitution with heavier isotopes such as deuterium ($^{2}H$) may afford certain therapeutic advantages that result from greater metabolic stability, for example increased in vivo half-life or reduced dosage requirements, and hence may be preferred in some circumstances. Substitution with positron emitting isotopes, such as $^{11}C$, $^{18}F$, $^{15}O$, and $^{13}N$ can be used in Positron Emission Tomography (PET) studies for examining substrate receptor occupancy.

In some embodiments, the thienotriazolodiazepine compound disclosed herein can exist in solvated as well as unsolvated forms with pharmaceutically acceptable solvents. It will be understood by those skilled-in the art that a solvate is a complex of variable stoichiometry formed by a solute (in this case, the thienotriazolodiazepine compound described herein) and a solvent. It is preferred that such solvents not interfere with the biological activity of the solute (the thienotriazolodiazepine compound). Examples of suitable solvents for solvate formation include water, methanol, dimethyl sulfoxide, ethanol and acetic acid. Suitably the solvent used is a pharmaceutically acceptable solvent. Suitably the solvent used is water. Described herein, pharmaceutically acceptable solvates of the thienotriazolodiazepine compound of the invention include ethanol solvate, a isopropanol solvate, a dioxolane solvate, a tetrahydrofuran solvate, a dimethyl sulfoxide solvate, tert-butanol solvate, 2-butanol solvate, dioxolane solvate, 1,3-Dimethyl-3,4,5,6-tetrahydro-2(1H)-pyrimidinone ("DMPU") solvate, 1,3-dimethylimidazolidinone ("DMI") solvate, and 1,3-dimethylimidazolidinone ("DMP") solvate, or mixtures thereof.

The thienotriazolodiazepine compound, described herein, contains a chiral center and exists as enantiomers. The enantiomer of the thienotriazolodiazepine compound may be designated in accordance with the Cahn-Ingold-Prelog convention, which assigns an *"R"* or *"S"descriptor* to each stereocenter (also sometimes referred to as a chiral center)so that the configuration of the entire molecule can be specified uniquely by including the descriptors in its systematic name.

In some embodiments, the symbol ------ may be used to denote a bond that may be a single, double or triple bond. Substituents around a carbon-carbon double bond are designated as being in the *"Z"* or *"E"* configuration wherein the terms *"Z"* and *"E"* are used in accordance with IUPAC standards. Unless otherwise specified, structures depicting double bonds encompass both the *"E"* and *"Z"* isomers. Substituents around a carbon-carbon double bond alternatively can be referred to as *"cis"* or *"trans,"* where *"cis"* represents substituents on the same side of the double bond and *"trans"* represents substituents on opposite sides of the double bond. The arrangement of substituents around a carbocyclic ring can also be designated as *"cis"* or *"trans."* The term *"cis"* represents substituents on the same side of the plane of the ring and the term *"trans"* represents substituents on opposite sides of the plane of the ring. Mixtures of compounds wherein the substituents are disposed on both the same and opposite sides of a plane of a ring are designated *"cis/trans"* or *"Z/E."*

In some embodiments, the thienotriazolodiazepine compound disclosed herein may exist in single or multiple crystalline forms or polymorphs. In one embodiment, a thienotriazolodiazepine compound disclosed herein comprises an amorphous form thereof. In one embodiment, a thienotriazolodiazepine compound disclosed herein comprises a single polymorph thereof. In another embodiment, a thienotriazolodiazepine compound disclosed herein comprises a mixture of polymorphs thereof. In another embodiment, the compound is in a crystalline form.

In some embodiments, the thienotriazolodiazepine compound disclosed herein may exist as a single enantiomers or in enatiomerically enriched forms. In one embodiment, a thienotriazolodiazepine compound disclosed herein exists in an entiomeric excess of more than 80%. In one embodiment, a thienotriazolodiazepine compound disclosed

herein exists in an entiomeric excess of more than 90%. In one embodiment, a thienotriazolodiazepine compound disclosed herein exists in an entiomeric excess of more than 98%. In one embodiment, a thienotriazolodiazepine compound disclosed herein exists in an entiomeric excess of more than 99%. In some embodiments, a thienotriazolodiazepine compound disclosed herein exists in an entiomeric excess selected from the group consisting of at least 10%, at least 25%, at least 50%, at least 75%, at least 90%, at least 95%, at least 98%, at least and at least 99% enantiomeric excess.

For a pair of enantiomers, enantiomeric excess (ee) of enantiomer *E1* in relation to enantiomer *E2* can be calculated using the following equation eq. (1):

$$\%\ \text{enantiomeric excess of } E1 = \frac{(E1 - E2)\text{x}100\%}{(E1 - E2)} \qquad \text{eq. (1)}$$

Relative amounts of *E1* and *E2* can be determined by chiral high performance liquid chromatography (HPLC), nuclear magnetic resonance (NMR) or any other suitable methods. In some embodiments, purity of an entiormeric compound may refer to the amount of the enantiomers *E1* and *E2,* relative to the amount of other materials, which may notably include by-products and/or unreacted reactants or reagents.

**III. Formulations:**

The compound described herein presents highly specific difficulties in relation to administration generally and the preparation of galenic compositions in particular, including the particular problems of drug bioavailability and variability in inter- and intra-patient dose response, necessitating development of a non-conventional dosage form with respect to the practically water-insoluble properties of the compound.

Previously, it had been found that the compound described herein could be formulated as a solid dispersion with the carrier ethyl acrylate-methyl methacrylate-trimethylammonioethyl methacrylate chloride copolymer (Eudragit RS, manufactured by Rohm) to provide an oral formulation that preferentially released the pharmaceutical ingredient in the lower intestine for treatment of inflammatory bowel diseases such as ulcerative colitis and Crohn's disease (US Patent Application 20090012064 A1, published Jan 8, 2009). It was found, through various experiments, including animal tests, that in inflammatory bowel diseases drug release in a lesion and a direct action thereof on the inflammatory lesion were more important than the absorption of the drug into circulation from the gastrointestinal tract.

It has now been unexpectedly found that the thienotriazolodiazepine compound described herein, pharmaceutically acceptable salts, hydrates, racemates, enantiomers isomers, and isotopically-labeled forms thereof, can be formulated as a solid dispersion with pharmaceutically acceptable polymers to provide an oral formulation that provides high absorption of the pharmaceutical ingredient into the circulation from the gastrointestinal tract for treatment of diseases other than inflammatory bowel diseases. Studies in both dogs and humans have confirmed high oral bioavailability of these solid dispersions compared with the Eudragit solid dispersion formulation previously developed for the treatment of inflammatory bowel disease.

Solid dispersions are a strategy to improve the oral bioavailability of poorly water soluble drugs.

The term "solid dispersion" as used herein refers to a group of solid products including at least two different components, generally a hydrophilic carrier and a hydrophobic drug, the thienotriazolodiazepine compound described herein. Based on the drug's molecular arrangement within the dispersion, six different types of solid dispersions can be distinguished. Commonly, solid dispersions are classified as simple eutectic mixtures, solid solutions, glass solution and suspension, and amorphous precipitations in a crystalline carrier. Moreover, certain combinations can be encountered, for example, in the same sample some molecules may be present in clusters while some are molecularly dispersed.

In one embodiment, the thienotriazolodiazepine compound described herein can be dispersed molecularly, in amorphous particles (clusters). In one embodiment, the carrier can be crystalline. In another embodiment, the carrier can be amorphous.

In one embodiment, the present invention provides a pharmaceutical composition comprising a solid dispersion of a thienotriazolodiazepine compound described herein, or a pharmaceutically acceptable salt, a hydrate, a racemate, an enantiomer, an isomer, or an isotopically-labeled form thereof; and a pharmaceutically acceptable polymer as described herein. In one embodiment, at least some portion of the thienotriazolodiazepine compound is homogeneously dispersed throughout the solid dispersion. In another embodiment, the thienotriazolodiazepine compound is homogeneously dispersed throughout the solid dispersion. In some embodiments, the solid dispersion exhibits a single inflection for the glass transition temperature (Tg). In some embodiments, the single Tg occurs between 130 °C to 140 °C. In other such embodiments, the single Tg occurs at about 135 °C. In some such embodiment, the solid dispersion was exposed to a relative humidity of 75 % at 40 °C for at least one month. In some embodiments, the solid dispersion exhibits an X-ray powder diffraction pattern substantially free of diffraction lines associated with crystalline thienotriazolodiazepine compound described herein. For the purpose of this application "substantially free" shall mean the absence of a diffraction

line, above the amorphous halo, at about 21°2-theta associated with crystalline thienotriazolodiazepine compound described herein.

In one embodiment, a pharmaceutical composition of the present invention comprises a solid dispersion of an amorphous form of a thienotriazolodiazepine compound described herein or a pharmaceutically acceptable salt, a hydrate, a racemate, an enantiomer, an isomer, or an isotopically-labeled form thereof and a pharmaceutically acceptable polymer, wherein the pharmaceutically acceptable polymer is hypromellose acetate succinate and wherein the weight ratio of thienotriazolodiazepine compound described herein to hypromellose acetate succinate ranges from 1:3 to 1:1. In one embodiment, at least some portion of the thienotriazolodiazepine compound is homogeneously dispersed throughout the solid dispersion. In another embodiment, the thienotriazolodiazepine compound is homogeneously dispersed throughout the solid dispersion. In some embodiments, the solid dispersion exhibits a single inflection for the glass transition temperature (Tg). In some embodiments, the single Tg occurs between 130 °C to 140 °C. In other such embodiments, the single Tg occurs at about 135 °C. In some such embodiment, the solid dispersion was exposed to a relative humidity of 75 % at 40 °C for at least one month. In some embodiments, the solid dispersion exhibits an X-ray powder diffraction pattern substantially free of diffraction lines associated with crystalline thienotriazolodiazepine compound described herein. For the purpose of this application "substantially free" shall mean the absence of a diffraction line, above the amorphous halo, at about 21 ° 2-theta associated with the crystalline thienotriazolodiazepine compound described herein.

In some embodiments, a pharmaceutical composition comprising a solid dispersion is prepared by spray drying.

In one embodiment, a pharmaceutical composition of the present invention comprises a spray dried solid dispersion of a thienotriazolodiazepine compound described herein or a pharmaceutically acceptable salt, a hydrate, a racemate, an enantiomer, an isomer, or an isotopically-labeled form thereof and a pharmaceutically acceptable polymer wherein the pharmaceutically acceptable polymer is hypromellose acetate succinate and wherein the weight ratio of thienotriazolodiazepine compound decribed herein to hypromellose acetate succinate ranges from 1:3 to 1:1. In one embodiment, at least some portion of the thienotriazolodiazepine compound is homogeneously dispersed throughout the solid dispersion. In another embodiment, the thienotriazolodiazepine compound is homogeneously dispersed throughout the solid dispersion. In some embodiments, the solid dispersion exhibits a single inflection for the glass transition temperature (Tg). In some embodiments, the single Tg occurs between 130 °C to 140 °C. In other such embodiments, the single Tg occurs at about 135 °C. In some such embodiment, the solid dispersion was exposed to a relative humidity of 75 % at 40 °C for at least one month. In some embodiments, the solid dispersion exhibits an X-ray powder diffraction pattern substantially free of diffraction lines associated with the crystalline thienotriazolodiazepine compound described herein. For the purpose of this application "substantially free" shall mean the absence of a diffraction line, above the amorphous halo, at about 21° 2-theta associated with the crystalline thienotriazolodiazepine compound described herein.

In one embodiment, a pharmaceutical composition of the present invention comprises a spray dried solid dispersion of an amorphous form of a thienotriazolodiazepine compound described herein or a pharmaceutically acceptable salt, a hydrate, a racemate, an enantiomer, an isomer, or an isotopically-labeled form thereof and a pharmaceutically acceptable polymer wherein the pharmaceutically acceptable polymer is hypromellose acetate succinate and wherein the weight ratio of the thienotriazolodiazepine compound described herein to hypromellose acetate succinate ranges from 1:3 to 1:1. In one embodiment, at least some portion of the thienotriazolodiazepine compound is homogeneously dispersed throughout the solid dispersion. In another embodiment, the thienotriazolodiazepine compound is homogeneously dispersed throughout the solid dispersion. In some embodiments, the solid dispersion exhibits a single inflection for the glass transition temperature (Tg). In some embodiments, the single Tg occurs between 130 °C to 140 °C. In some such embodiment, the solid dispersion was exposed to a relative humidity of 75 % at 40 °C for at least one month. In other such embodiments, the single Tg occurs at about 135 °C. In some embodiments, the solid dispersion exhibits an X-ray powder diffraction pattern substantially free of diffraction lines associated with the crystalline thienotriazolodiazepine compound described herein. For the purpose of this application "substantially free" shall mean the absence of a diffraction line, above the amorphous halo, at about 21° 2-theta associated with the crystalline thienotriazolodiazepine compound described herein.

In one embodiment, a pharmaceutical composition of the present invention comprises a solid dispersion of an amorphous form of a thienotriazolodiazepine compound (1-1) or a pharmaceutically acceptable salt, a hydrate, a racemate, an enantiomer, an isomer, or an isotopically-labeled form thereof; and a pharmaceutically acceptable polymer wherein the pharmaceutically acceptable polymer is HPMCAS and wherein the dispersion has compound (1-1) and HPMCAS in a weight ratio of 1:3 to 1:1. In one embodiment, at least some portion of the thienotriazolodiazepine compound is homogeneously dispersed throughout the solid dispersion. In another embodiment, the thienotriazolodiazepine compound is homogeneously dispersed throughout the solid dispersion. In one embodiment, the solid dispersion is spray dried. In some embodiments, the solid dispersion exhibits a single inflection for the glass transition temperature (Tg). In some embodiments, the single Tg occurs between 130 °C to 140 °C. In other such embodiments, the single Tg occurs at about 135 °C. In some such embodiment, the solid dispersion was exposed to a relative humidity of 75 % at 40 °C

for at least one month. In some embodiments, the solid dispersion exhibits an X-ray powder diffraction pattern substantially free of diffraction lines associated with crystalline thienotriazolodiazepine compound (1-1). For the purpose of this application "substantially free" shall mean the absence of a diffraction line, above the amorphous halo, at about 21° 2-theta associated with crystalline thienotriazolodiazepine compound (1-1).

The solid dispersions of the invention, described herein, exhibit especially advantageous properties when administered orally. Examples of advantageous properties of the solid dispersions include consistent and high level of bioavailability when administered in standard bioavailability trials in animals or humans. The solid dispersions of the invention can include a solid dispersion comprising the thienotriazolodiazepine compound described herein and a polymer and additives. In some embodiments, the solid dispersions can achieve absorption of the thienotriazolodiazepine compound described herein into the bloodstream that cannot be obtained by merely admixing the thienotriazolodiazepine compound with additives since the thienotriazolodiazepine compound drug has negligible solubility in water and most aqueous media. The bioavailability, of the thienotriazolodiazepine compound described herein, i.e. thienotriazolodiazepine compound (1-1), may be measured using a variety of in vitro and/or in vivo studies. The in vivo studies may be performed using rats, dogs or humans.

The bioavailability may be measured by the area under the curve (AUC) value obtained by plotting a serum or plasma concentration, of the thienotriazolodiazepine compound described herein or thienotriazolodiazepine compound (1-1), along the ordinate (Y-axis) against time along the abscissa (X-axis). The AUC value of the thienotriazolodiazepine compound described herein or thienotriazolodiazepine compound (1-1) from the solid dispersion, is then compared to the AUC value of an equivalent concentration of crystalline thienotriazolodiazepine compound described herein or crystalline thienotriazolodiazepine compound (1-1) without polymer. In some embodiments, the solid dispersion provides an area under the curve (AUC) value, when administered orally to a dog, that is selected from: at least 0.4 times, 0.5 times, 0.6 time, 0.8 time, 1.0 times, a corresponding AUC value provided by a control composition administered intravenously to a dog, wherein the control composition comprises an equivalent quantity of a crystalline thienotriazolodiazepine compound of Formula I.

The bioavailability may be measured by in vitro tests simulating the pH values of a gastric environment and an intestine environment. The measurements may be made by suspending a solid dispersion of the thienotriazolodiazepine compound described herein, i.e. thienotriazolodiazepine compound (1-1), in an aqueous in vitro test medium having a pH between 1.0 to 2.0, and the pH is then adjusted to a pH between 5.0 and 7.0, in a control in vitro test medium. The concentration of the amorphous thienotriazolodiazepine compound described herein, i.e. amorphous thienotriazolodiazepine compound (1-1), may be measured at any time during the first two hours following the pH adjustment. In some embodiments, the solid dispersion provides a concentration, of the amorphous thienotriazolodiazepine compound described herein, i.e. amorphous thienotriazolodiazepine compound (1-1), in an aqueous in vitro test medium at pH between 5.0 to 7.0 that is selected from: at least 5-fold greater, at least 6 fold greater, at least 7 fold greater, at least 8 fold greater, at least 9 fold greater or at least 10 fold greater, compared to a concentration of a crystalline thienotriazolodiazepine compound described herein, i.e. crystalline thienotriazolodiazepine compound (1-1), without polymer.

In other embodiments, the concentration of the amorphous thienotriazolodiazepine compound described herein, i.e. amorphous thienotriazolodiazepine compound (1-1), from the solid dispersion placed in an aqueous in vitro test medium having a pH of 1.0 to 2.0, is: at least 40%, at least 50% higher, at least 60 %, at least 70 %; at least 80 %, than a concentration of a crystalline thienotriazolodiazepine compound described herein without polymer.

In other embodiments, a concentration of the amorphous thienotriazolodiazepine compound described herein, i.e. amorphous thienotriazolodiazepine compound (1-1), from the solid dispersion, is: at least 40%, at least 50% higher, at least 60 %, at least 70 %; at least 80 %, compared to a concentration of the thienotriazolodiazepine compound described herein, from a solid dispersion of the thienotriazolodiazepine compound described herein and a pharmaceutically acceptable polymer selected from the group consisting of: hypromellose phthalate and ethyl acrylate-methyl methacrylate-trimethylammonioethyl methacrylate chloride copolymer, wherein each solid dispersion was placed in an aqueous in vitro test medium having a pH of 1.0 to 2.0.

In some embodiments, the solid dispersions, described herein, exhibit stability against recrystallization of the thienotriazolodiazepine compound described herein, i.e. the thienotriazolodiazepine compound (1-1), when exposed to humidity and temperature over time. In one embodiment, the concentration of the amorphous thienotriazolodiazepine compound described herein, i.e. thienotriazolodiazepine compound (1-1) which remains amorphous is selected from: at least 90 %, at least 91%, at least 92%, at least 93%, at least 94%, at least 95%, at least 96%, at least 97%, at least 98% and at least 99%

**IV. Dosage Forms:**

Suitable dosage forms that can be used with the solid dispersions of the present invention include capsules, tablets, mini-tablets, beads, beadlets, pellets, granules, granulates, and powder. Suitable dosage forms may be coated, for example using an enteric coating. Suitable coatings may comprise cellulose acetate phthalate, hydroxypropylmethylcellulose (HPMC), hydroxypropylmethylcellulose phthalate, a polymethylacrylic acid copolymer, or hydroxylpropylmethylcellulose acetate succinate (HPMCAS). In some embodiments, certain combinations can be encountered, for example, in the same sample some molecules of the thienotriazolodiazepine of the present invention may be present in clusters while some are molecularly dispersed with a carrier.

In some embodiments, the solid dispersions of the invention may be formulated as tablets, caplets, or capsules. In one some embodiments, the solid dispersions of the invention may be formulated as mini-tablets or pour-into-mouth granules, or oral powders for constitution. In some embodiments, the solid dispersions of the invention are dispersed in a suitable diluent in combination with other excipients (i.e., re-crystallization/precipitation inhibiting polymers, taste-masking components, etc) to give a ready-to-use suspension formulation. In some embodiments, the solid dispersions of the invention may be formulated for pediatric treatment.

In one embodiment, the pharmaceutical composition of the present invention is formulated for oral administration. In one embodiment, the pharmaceutical composition comprises a solid dispersion, according to the various embodiments described herein, comprising a thienotriazolodiazepine compound described herein or a pharmaceutically acceptable salt, a hydrate, a racemate, an enantiomer, an isomer, or an isotopically-labeled form thereof; and a polymer carrier. In one embodiment, the pharmaceutical composition further includes one or more additives such as disintegrants, lubricants, glidants, binders, and fillers.

Examples of suitable pharmaceutically acceptable lubricants and pharmaceutically acceptable glidants for use with the pharmaceutical composition include colloidal silica, magnesium trisilicate, starches, talc, tribasic calcium phosphate, magnesium stearate, aluminum stearate, calcium stearate, magnesium carbonate, magnesium oxide, polyethylene glycol, powdered cellulose, glyceryl behenate, stearic acid, hydrogenated castor oil, glyceryl monostearate, and sodium stearyl fumarate.

Examples of suitable pharmaceutically acceptable binders for use with the pharmaceutical composition include starches; celluloses and derivatives thereof, e.g., microcrystalline cellulose (e.g., AVICEL PH from FMC), hydroxypropyl cellulose, hydroxyethyl cellulose, and hydroxylpropylmethylcellulose (HPMC, e.g., METHOCEL from Dow Chemical); sucrose, dextrose, corn syrup; polysaccharides; and gelatin.

Examples of suitable pharmaceutically acceptable fillers and pharmaceutically acceptable diluents for use with the pharmaceutical composition include confectioner's sugar, compressible sugar, dextrates, dextrin, dextrose, lactose, mannitol, microcrystalline cellulose (MCC), powdered cellulose, sorbitol, sucrose, and talc.

In some embodiments, excipients may serve more than one function in the pharmaceutical composition. For example, fillers or binders may also be disintegrants, glidants, anti-adherents, lubricants, sweeteners and the like.

In some embodiments, the pharmaceutical compositions of the present invention may further include additives or ingredients, such as antioxidants (e.g., ascorbyl palmitate, butylated hydroxylanisole (BHA), butylated hydroxytoluene (BHT), $\alpha$-tocopherols, propyl gallate, and fumaric acid), antimicrobial agents, enzyme inhibitors, stabilizers (e.g., malonic acid), and/or preserving agents.

Generally, the pharmaceutical compositions of the present invention may be formulated into any suitable solid dosage form. In some embodiments, the solid dispersions of the invention are compounded in unit dosage form, e.g., as a capsule, or tablet, or a multi-particulate system such as granules or granulates or a powder, for administration.

In one embodiment, a pharmaceutical compositions includes a solid dispersion of a thienotriazolodiazepine compound described herein, according to the various embodiments of solid dispersions described herein, and hydroxypropylmethylcellulose acetate succinate (HPMCAS), wherein the thienotriazolodiazepine compound is amorphous in the solid dispersion and has a thienotriazolodiazepine compound to hydroxypropylmethylcellulose acetate succinate (HPMCAS), weight ratio of 1:3 to 1:1; 45 -50 wt. % of lactose monohydrate; 35-40 wt. % of microcrystalline cellulose; 4-6 wt. % of croscarmellose sodium; 0.8-1.5 wt. % of colloidal silicon dioxide; and 0.8-1.5 wt. % of magnesium stearate.

**V. Dosage:**

In one embodiment, the present invention provides a pharmaceutical composition that maybe formulated into any suitable solid dosage form. In one embodiment, a pharmaceutical composition in accordance with the present invention comprises one or more of the various embodiments of the thienotriazolodiazepine compound of the invention as described herein in a dosage amount ranging from about 10 mg to about 100 mg. In one embodiment, the pharmaceutical composition of the present invention includes one or more of the various embodiments of the thienotriazolodiazepine compound of the invention as described herein in a dosage amount selected from the group consisting of from about 10 mg to about 100 mg, about 10 mg to about 90 mg, about 10 mg to about 80 mg, about 10 mg to about 70 mg, about 10 mg to about 60 mg, about 10 mg to about 50 mg, about 10 mg to about 40 mg, about 10 mg to about 30 mg, and about 10 mg to about 20 mg. In one embodiment, the pharmaceutical composition of the present invention includes one or more of the various embodiments of the thienotriazolodiazepine compound of the invention as described herein in a dosage amount selected from the group consisting of about 10 mg, about 50 mg, about 75 mg, about 100 mg.

Such unit dosage forms are suitable for administration 1 to 5 times daily depending on the particular purpose

of therapy, the phase of therapy, and the like. In one embodiment, the dosage form may be administered to a subject in need thereof at least once daily for at least two successive days. In one embodiment, the dosage form may be administered to a subject in need thereof at least once daily on alternative days. In one embodiment, the dosage form may be administered to a subject in need thereof at least weekly and divided into equal and/or unequal doses. In one embodiment, the dosage form may be administered to a subject in need thereof weekly, given either on three alternate days and/or 6 times per week. In one embodiment, the dosage form may be administered to a subject in need thereof in divided doses on alternate days, every third day, every fourth day, every fifth day, every sixth day and/or weekly. In one embodiment, the dosage form may be administered to a subject in need thereof two or more equally or unequally divided doses per month.

The dosage form used, e.g., in a capsule, tablet, mini-tablet, beads, beadlets, pellets, granules, granulates, or powder may be coated, for example using an enteric coating. Suitable coatings may comprise cellulose acetate phthalate, hydroxypropylmethylcellulose (HPMC), hydroxypropylmethylcellulose phthalate, a polymethylacrylic acid copolymer, or hydroxylpropylmethylcellulose acetate succinate (HPMCAS).

**VI. Process:**

The thienotriazolodiazepine compound disclosed herein can exist as free base or as acid addition salt can be obtained according to the procedures described in US Patent Application Publication No. 2010/0286127 or in the present application. The individual enantiomer of the thienotriazolodiazepine compound of the present invention can be prepared synthetically from commercially available starting materials that contain asymmetric or stereogenic centers, or by preparation of racemic mixtures followed by resolution methods well known to those of ordinary skill in the art. These methods of resolution are exemplified by (1) attachment of a mixture of enantiomers to a chiral auxiliary, separation of the resulting mixture of diastereomers by recrystallization or chromatography and liberation of the optically pure product from the auxiliary, (2) salt formation employing an optically active resolving agent, (3) direct separation of the mixture of optical enantiomers on chiral liquid chromatographic columns or (4) kinetic resolution using stereoselective chemical or enzymatic reagents. Racemic mixtures can also be resolved into their component enantiomers by well-known methods, such as chiral-phase gas chromatography or crystallizing the compound in a chiral solvent.

If desired, a particular enantiomer of the thienotriazolodiazepine compound disclosed herein may be prepared by asymmetric synthesis, or by derivation with a chiral auxiliary, where the resulting diastereomeric mixture is separated and the auxiliary group cleaved to provide the pure desired enantiomers. Alternatively, where the molecule contains a basic functional group, such as amino, or an acidic functional group, such as carboxyl, diastereomeric salts are formed with an appropriate optically-active acid or base, followed by resolution of the diastereomers, thus formed by fractional crystallization or chromatographic means well known in the art, and subsequent recovery of the pure enantiomers. Various methods well known in the art may be used to prepare the thienotriazolodiazepine compound described herein with an enantiomeric excess of generally more than about 80%. Advantageously, preferred enantiomeric excess is of more than 80%, preferably of more than 90%, more preferably of more than 95%, and most preferably of 99% and more.

The solid dispersions of the present invention can be prepared by a number of methods, including by melting and solvent evaporation. The solid dispersions of the present invention can also be prepared according to the procedures described in: Chiou WL, Riegelman S: "Pharmaceutical applications of solid dispersion systems", J. Pharm. Sci. 1971; 60:1281-1302; Serajuddin ATM: "Solid dispersion of poorly water-soluble drugs: early promises, subsequent problems, and recentbreakthroughs", J. Pharm. Sci. 1999; 88:1058-1066; Leuner C, Dressman J: "Improving drug solubility for oral delivery using solid dispersions", Eur. J. Pharm. Biopharm. 2000; 50:47-60; and Vasconcelos T, Sarmento B, Costa P: "Solid dispersions as strategy to improve oral bioavailability of poor water soluble drugs", Drug Discovery Today 2007; 12:1068-1075.

In one embodiment, solid dispersions of the present invention is prepared by a melting process. In one embodiment, the melting process comprises melting one or more of the various embodiments of the thienotriazolodiazepine described herein within a carrier. In one embodiment, the melting process includes cooling a melted compound of the present invention and a carrier. In one embodiment, the melting process comprises pulverization of the melted compound and the carrier. In one embodiment, a melted compound of the present invention and a carrier are pulverized following the cooling step.

In some embodiments in which the thienotriazolodiazepine described herein or a pharmaceutically acceptable salt, a hydrate, a racemate, an enantiomer, an isomer, or an isotopically-labeled form thereof and the carrier are incompatible, a surfactant may be added during the melting step to prevent formation of two liquid phases or a suspension in the heated mixture. In some embodiments, one or more of the various embodiments of the thienotriazolodiazepine described herein is suspended in a previously melted carrier, instead of using both drug and carrier in the melted state, thereby reducing the process temperature. In one embodiment, melted drug and carrier mixture is cooled an ice bath agitation. In one embodiment, melted drug and carrier mixture is cooled and solidified by spray cooling (alternatively spray congealing).

In one embodiment, melted drug and carrier mixture is cooled and solidified by forming the melt into particles by spraying the melt into a cooling chamber through which ambient or cooled, low temperature air is passing. In one embodiment, melted drug and carrier mixture is cooled and solidified by atomization and re-solidification of the molten dispersion in a suitable fluid bed processor. In one embodiment, melted drug and carrier mixture is cooled and solidified by melt-granulation in a heatable high-shear mixer.

In some embodiments, hot-stage extrusion or melt agglomeration may be used to avoid melting limitations of the drug. Hot-stage extrusion consists of the extrusion, at high rotational speed, of the drug and carrier, previously mixed, at melting temperature for a short period of time; the resulting product is collected after cooling at room temperature and milled.

In one embodiment, one or more of the various embodiments of the thienotriazolodiazepine described herein is processed at a reduced processing temperature to avoid degradation of any thermally labile compound. In one embodiment, the reduced processing temperature is achieved by associating a hot-stage extrusion with a temporary plasticizer such as carbon dioxide. In one embodiment, melt agglomeration is used in the preparation of solid dispersions in accordance with the present invention in conventional high shear mixers or in a rotary processors. In one embodiment, the solid dispersion in accordance with the present invention is prepared by adding a molten carrier containing a thienotriazolodiazepine compound in accordance with the present invention to a heated excipient. In one embodiment, the solid dispersion in accordance with the present invention is prepared by adding by adding a molten carrier to a heated mixture of the thienotriazolodiazepine in accordance with the present invention and one or more excipients. In one embodiment, the solid dispersion in accordance with the present invention is prepared by heating a mixture of a thienotriazolodiazepine compound in accordance with the present invention, a carrier and one or more excipients to a temperature within or above the melting range of the carrier.

In some embodiments, a one or more of the various embodiments for the formulation of the thienotriazolodiazepine, described herein, is prepared by a solvent evaporation method. In one embodiment, the solvent evaporation method comprises solubilization of a thienotriazolodiazepine compound, described herein, carrier in a volatile solvent that is subsequently evaporated. In one embodiment, the volatile solvent may one or more excipients. In one embodiment, the one or more excipients include anti-sticking agents, inert fillers, surfactants wetting agents, pH modifiers and additives. In one embodiment, the excipients may dissolved or in suspended or swollen state in the volatile solvent.

In one embodiment, preparation of solid dispersions in accordance with the present invention includes drying one or more excipients suspended in a volatile solvent. In one embodiment, the drying includes vacuum drying, slow evaporation of the volatile solvent at low temperature, use of a rotary evaporator, spray-drying, spray granulation, freeze-drying, or use of supercritical fluids.

In one embodiment, spray drying preparation of a formulation for the thienotriazolodiazepine composition, described herein, is used which involves atomization of a suspension or a solution of the composition into small droplets, followed by rapid removal solvent from the formulation. In one embodiment, preparation of a formulation in accordance with the present invention involves spray granulation in which a solution or a suspension of the composition in a solvent is sprayed onto a suitable chemically and/or physically inert filler, such as lactose or mannitol. In one embodiment, spray granulation of the solution or the suspension of the composition is achieved via two-way or three-way nozzles.

In some embodiments, preparation of solid dispersions in accordance with the present invention includes use of supercritical fluids. The term "supercritical fluids" refers to substances existing as a single fluid phase above their critical temperature and critical pressure. In one embodiment, preparation of a formulation, in accordance with the present invention, includes use a supercritical carbon dioxide fluid. In one embodiment, preparation of a formulation, in accordance with the present invention, using the supercritical fluid technique comprises dissolving a thienotriazolodiazepine compound, described herein, and carrier in a common solvent that is introduced into a particle formation vessel through a nozzle, simultaneously with carbon dioxide; and spraying the solution to allow the solvent be rapidly extracted by the supercritical fluid, thereby resulting in the precipitation of solid dispersion particles on the walls of the vessel.

In some embodiments, preparation of solid dispersions in accordance with the present invention includes use of a co-precipitation method. In one embodiment, a non-solvent is added dropwise to a thienotriazolodiazepine composition, described herein, and a carrier solution, under constant stirring. In one embodiment, the thienotriazolodiazepine composition, described herein, and the carrier are co-precipitated to form microparticles during the addition of the non-solvent. In one embodiment, the resulting microparticles are filtered and dried to provide the desired solid dispersion.

The proportion of the thienotriazolodiazepine compound described herein and polymeric carrier(s) to be mixed is not particularly limited, as long as it can improve the bioavailability of the compound described herein and varies depending on the kind of polymer.

The invention is illustrated in the following examples.

**VII. Examples:**

Example 1: *in vitro* screening of solid dispersions of compound (1-1)

Ten solid dispersions were prepared using compound (1-1) and one of five polymers, including hypromellose acetate succinate (HPMCAS-M), hypromellose phthalate (HPMCP-HP55), polyvinylpyrrolidone (PVP), PVP-vinyl acetate (PVP-VA), and Euragit L100-55, at both 25% and 50% of compound (1-1) loading, for each polymer. Solid dispersions were prepared by a solvent evaporation method, using spray-drying followed by secondary drying in a low-temperature convection oven. The performance of each solid dispersion was assessed via a non-sink dissolution performance test which measured both the total amount of drug and the amount of free drug present in solution over time. Non-sink dissolution was chosen because it best represents the in vivo situation for low soluble compounds. This test included a "gastric transfer" of dispersion from gastric pH (0.1N NaCl, pH 1.0) to intestinal pH (FaFSSIF, pH 6.5) approximately 30 to 40 minutes after the introduction of dispersion to the test medium, simulating *in vivo* conditions. [FaFSSIF is Fasted State Simulated Intestinal Fluid, comprised of 3 mM sodium taurocholate, 0.75 mM lechithin, 0.174 g NaOH pellets, 1.977 g $NaH_2PO_4 \bullet H_2O$, 3.093 g NaCl, and purified water qs 500 mL.] The amount of dissolved drug was quantified using a high-performance liquid chromatrography (HPLC) method and an Agilent 1100 series HPLC. The dissolution profiles of the formulations (Figures 1A-1J) showed large increases in drug solubility in all dispersion candidates relative to the unformulated compound in the same media. Of the solid dispersions, the 25% compound (1-1) in PVP, 25% compound (1-1) in HPMCAS-M, and 50% compound (1-1) in HPMCAS-M dispersions provided enhanced oral absorption as compared to the unformulated compound, based on finding higher levels of free drug released at intestinal pH.

Example 2: *in vivo* screening of solid dispersions of compound (1-1)

The solid dispersions of compound (1-1), namely the 25% compound (1-1) in PVP, 25% compound (1-1) in HPMCAS-MG, and 50% compound (1-1) in HPMCAS-M dispersions, were prepared at larger scale for *in vivo* studies. Each formulation was assessed in the in vitro dissolution test described in Example 1. To ensure that these dispersions were both amorphous and homogeneous, each dispersion was assessed by powder x-ray diffraction (PXRD) and modulated differential scanning calorimetry (mDSC). The x-ray diffractomer was a Bruker D-2 Phaser. Additionally, to understand the effect of water on the glass transition temperature (Tg) for each dispersion, mDSC was performed on samples first equilibrated at a set relative humidity (i.e., 25%, 50%, and 75% RH) for at least 18 hours. [Water can act as a plasticizer for solid dispersions and the hygroscopicity of the system due to the active compound or polymer can affect the amount of water uptake by these systems.]

The non-sink dissolution results (Figures 2A-2C) were comparable to those found for the dispersions in Example 1. PXRD results (Figure 3) showed no evidence of crystalline compound in any of the dispersions and mDSC results (Figures 4A-4C) showed a single glass transition temperature (Tg) for each dispersion, indicating that each dispersion was homogeneous. An inverse relationship between Tg and relative humidity was observed for each (Figure 5). Notably, for the 25% compound (1-1) in PVP solid dispersion equilibrated at 75% RH, there appeared to be two Tgs, indicating that phase separation was occurring, and this dispersion also showed a melt event at 75% RH, suggesting that crystallization occurred during the RH equilibration (Figure 6). This finding suggests that the 25% compound (1-1) in PVP dispersion may be less stable than the HPMCAS-M dispersions.

To assess the bioavailability of the three dispersions, groups of male beagle dogs (three per group) were given a 3 mg/kg dose of an aqueous suspension of solid dispersion of compound (1-1) administered by oral gavage or a 1 mg/kg dose of compound (1-1) dissolved in water:ethanol:polyethylene glycol (PEG) 400 (60:20:20) and administered as an intravenous bolus into the cephalic vein. Blood samples were collected from the jugular vein of each animal at 0 (pre-dose), 5, 15, and 30 minutes and 1, 2, 4, 8, 12, and 24 hours following intravenous administration and at 0 (pre-dose), 15 and 30 minutes and 1, 2, 4, 8, 12, and 24 hours following oral gavage administration. The amount of compound (1-1) present in each sample was detected using a qualified LC-MS/MS method with a lower limit of quantification of 0.5 ng/mL. The area under the plasma concentration-time curve (AUC) was determined by use of the linear trapezoidal rule up to the last measurable concentration without extrapolation of the terminal elimination phase to infinity. The elimination half-life ($t_{1/2}$) was calculated by least-squares regression analysis of the terminal linear part of the log concentration-ime curve. The maximum plasma concentration ($C_{max}$) and the time to $C_{max}$ ($t_{max}$) were derived directly from the plasma concentration data. The oral bioavailability (F) was calculated by dividing the dose normalized AUC after oral administration by the dose normalized AUC after intravenous administration and reported as percentages (%). Results, summarized in Table 1 below, gave mean oral bioavailabilities of the 25% compound (1-1) in PVP, 25% compound (1-1) in HPMCAS-M, and 50% compound (1-1) in HPMCAS-M solid dispersions of 58%, 49%, and 74%, respectively.

Table 1: pharmacokinetic parameters of compound (1-1) after oral (po) and intravenous (iv) administrations to dogs (the values are averages from three dogs)

| Compound (1-1) formulation | Dose & Route | $C_{max}$ (ng/L) | $t_{max}$ (hr) | AUC (ng•min/mL) | $t_{1/2}$ (hr) | F (%) |
|---|---|---|---|---|---|---|
| Solution in water:ethanol: PEG400 (60:20:20) | 1 mg/kg IV | 769 | 0.083 | 53,312 | 1.5 | ---- |
| Aqueous suspension of 25% compound (1-1)/PVP solid dispersion | 3 mg/kg PO | 487 | 1.0 | 93,271 | 1.6 | 58 |
| Aqueous suspension of 25% compound (1-1)/HPMCAS-M solid dispersion | 3 mg/kg PO | 228 | 0.5 | 78,595 | 2.0 | 49 |
| Aqueous suspension of 50% compound (1-1)/HPMCAS-M solid dispersion | 3 mg/kg PO | 371 | 1.0 | 118,174 | 1.5 | 74 |
| AUC: area under the plasma concentration-time curve; $C_{max}$: maximum plasma concentration; F: bioavailability; HPMCAS: hypromellose acetate sodium; IV: intravenous; PEG: polyethylene glycon; PO; *per os,* oral; PVP: polyvinylpyrrolidone; $t_{max}$: time of $C_{max}$; $t_{1/2}$: plasma elimination half-life | | | | | | |

Example 3: preparation and clincial use of capsules containing a solid dispersion of compound (1-1)

A gelatin capsule of 10 mg strength was prepared for initial clinical studies in patients with hematologic malignancies. Based on results of *in vitro* and *in vivo* testing of solid dispersions of compound (1-1), as described in Examples 1 and 2, a 50% compound (1-1) in HPMCAS-M solid dispersion was selected for capsule development. Capsule development was initiated targeting a fill weight of 190 mg in a size 3 hard gelatin capsule, as this configuration would potentially allow increasing the capsule strength by filling a larger size capsule while maintaining the pharmaceutical composition. Based on experience, four capsule formulations were designed with different amounts of disintegrant and with and without wetting agent. Since all four formulations showed similar disintegration test and dissolution test results, the simplest formulation (without wetting agent and minimum disintegrant) was selected for manufacturing. Manufacturing process development and scale-up studies were performed to confirm the spray drying process and post-drying times for the solid dispersion; blending parameters; roller compaction and milling of the blend to achieve target bulk density of approximately 0.60 g/cc; and capsule filling conditions.

Crystalline compound (1-1) and the polymer hypromellose actate succinate (HPMCAS-M) were dissolved in acetone and spray-dried to produce solid dispersion intermediate (SDI) granules containing a 50% compound (1-1) loading. The SDI was shown by PXRD analysis to be amorphous and by mDSC analysis to be homogeneous (i.e., single Tg under ambient conditions). The 50% compound (1-1) in HPMCAS-M solid dispersion (1000 g) and excipients, including microcrystalline cellulose filler-binder (4428 g), croscarmellose sodium disintegrant (636 g), colloidal silicon dioxide dispersant/lubricant 156 g), magnesium stearate dispersant/lubricant (156 g), and lactose monohydrate filler (5364 g) were blended in stages in a V-blender. The blend was them compacted and granulated to obtain a bulk density of approximately 0.6 g/mL. The blend was dispensed into size 3 hard gelatin capsules (target fill weight: 190 mg) using an automated filling machine and finished capsules were polished using a capsule polisher machine.

Pharmacokinetic assessments were performed following oral dosing of 10 mg capsules containing the 50% compound (1-1) in HPMCAS solid dispersion and results were compared with pharmacokinetic assessments performed following oral dosing of administration of 4 x 10 mg capsules containing the Eudragit solid dispersion of compound (1-1) to healthy volunteers

A comparison of the two pharmaceutical compositions is provided in Tables 2A and 2B below. The Eudragit formulation previously was described in Example 5 in US Patent Application 2009/0012064 A1, published January 8, 2009. That application noted that the Eudragit solid dispersion formulation was made by dissolving and/or dispersing the thienotriazolodiazepine of formula (A) and coating excipients, including ammonio methacrylate copolymer type B (Eudragit RS), methacrylic acid copolymer type C (Eudragit L100-55), talc, and magnesium aluminosilicate, in a mixture of water and ethanol. This heterogeneous mixture then was applied to microcrystalline cellulose spheres (Nonpareil 101, Freund) using a centrifugal fluidizing bed granulator to produce granules that were dispensed into size 2 hydroxypropyl methylcellulose capsules.

In both clinical studies, blood levels of compound (1-1) were determined using validated LC-MS/MS methods and pharmacokinetic analyses were performed based on plasma concentrations of compound (1-1) measured at various time points over 24 hours after capsule administration. Results, summarized in Table 3 below, showed that the HPMCAS-M solid dispersion formulation had over 3-fold higher bioavailability in humans than the Eudragit solid dispersion formulation based on AUCs (924*4/1140, adjusting for difference in doses administered). Additionally, based on the observed $T_{max}$, the HPMCAS formulation is more rapidly absorbed than the Eudragit formulation ($T_{max}$ of 1 h vs 4-6 h). The marked improvement in systemic exposure with the HPMCAS-M solid dispersion formulation is unexpected.

In table 2A Compound of formula (II) refers to Compound (1-1).

Table 2A: solid dispersion capsules of compound (1-1) for clinical use

| pharmaceutical composition containing 50% HPMCAS solid dispersion of compound (1-1 10 mg strength, size 3 hard gelatin capsule | | | |
|---|---|---|---|
| Ingredient | Function | Capsule Content | |
| | | mg | Wt % |
| Compound of formula (II) | active agent | 10.0* | 5.56 |
| Hypromellose acetate succinate (HPMCAS-M) | carrier for solid dispersion | 10.0 | 5.56 |
| Lactose monohydrate | filler | 85.0 | 47.22 |
| Microcrystalline cellulose | filler-binder | 70.0 | 38.89 |
| Croscarmellose sodium | disintegrant | 10.0 | 5.56 |
| Collidal silicon dioxide | dispersant/lubricant | 2.5 | 1.39 |
| Magnesium stearate | dispersant/lubricant | | |
| | Total | 190.0 | 100.0 |

Table 2B: pharmaceutical composition containing Eudragit L100-55solid dispersion of compound (1-1): 10 mg strength, size 2 hard gelatin capsule

| Ingredient | Function | Capsule Content | |
|---|---|---|---|
| | | mg | Wt % |
| Compound (1-1) | active agent | 10.0* | 3.8 |
| Core: | | | |
| Microcrystalline cellulose spheres (Nonpareil 101, Freund, Inc) | vehicle | 100.0 | 38.5 |
| Compound/polymer layer: | | | |
| Ammonio methacrylate copolymer, type B (NF. PhEur) (Edragit RS, Evonik) | coating agent | 10.8 | 4.2 |
| Methacrylic acid copolymer, type C (NF)/ Methacrylic acid-ethyl acrylate copolymer (1:1) type A (PhEur) (Eudragit L100-55, Evonik) | coating agent | 25.2 | 9.7 |
| Talc | coating agent | 88.2 | 33.9 |
| Magnesium aluminometasilicate (Neuslin, Fuji Chemical) | coating agent | 20.0 | 7.7 |
| Triethyl citrate | plasticizer | 5.0 | 1.9 |
| Silicon dioxide | fluidizing agent | 0.8 | 0.3 |
| | | 260.0 | 100.0 |
| * as anhydrate | | | |

Table 3: pharmacokinetic parameters following oral administration of solid dispersions of compound (1-1) to humans

| Compound (1-1) formulation | # Patients | Dose and Route | $C_{max}$ (ng/mL) | $T_{max}$ (hr) | $AUC_{0-24h}$ (ng•h/mL) |
|---|---|---|---|---|---|
| Eudragit solid dispersion formulation | 7 | 40 mg PO | 83 | 4 to 6 | 1140 |
| 50% HMPCAS-M solid dispersion formulation | 7 | 10 mg PO | 286 | 1 | 925 |
| $AUC_{0-24h}$: area under the OTX015 plasma concentration vs. time curve over 24 hours<br>$C_{max}$: maximum concentration in plasma<br>hr: hour<br>HPMCAS: hypromellose acetate succinate<br>mL: milliliter<br>ng: nanogram<br>PO: *per os,* oral<br>$T_{max}$: time of $C_{max}$ | | | | | |

Example 4. Oral exposure in the rat

The oral bioavailability of three formulations of solid dispersions of compound (1-1) was determined in rats. The three dispersions chosen were the 25% dispersion of compound (1-1) in PVP, the 25% dispersion of compound (1-1) in HPMCAS-MG, and the 50% dispersion of compound (1-1) in HPMCAS-MG. The animals used in the study were Specific Pathogen Free (SPF) Hsd:Sprague Dawley rats obtained from the Central Animal Laboratory at the University of Turku, Finland. The rats were originally purchased from Harlan, The Netherlands. The rats were female and were ten weeks of age, and 12 rats were used in the study. The animals were housed in polycarbonate Makrolon II cages (three animals per cage), the animal room temperature was 21 +/-3 °C, the animal room relative humidity was 55 +/- 15%, and the animal room lighting was artificial and was cycled for 12 hour light and dark periods (with the dark period between 18:00 and 06:00 hours). Aspen chips (Tapvei Oy, Estonia) were used for bedding, and bedding was changed at least once per week. Food and water was provided prior to dosing the animals but was removed during the first two hours after dosing.

The oral dosing solutions containing the 25% dispersion of compound (1-1) in PVP, the 25% dispersion of compound (1-1) in HPMCAS-MG, and the 50% dispersion of compound (1-1) in HPMCAS-MG were prepared by adding a pre-calculated amount of sterile water for injection to containers holding the dispersion using appropriate quantities to obtain a concentration of 0.75 mg/mL of compound (1-1). The oral dosing solutions were subjected to vortex mixing for 20 seconds prior to each dose. The dosing solution for intravenous administration contained 0.25 mg/mL of compound (1-1) and was prepared by dissolving 5 mg of compound (1-1) in a mixture containing 4 mL of polyethylene glycol with an average molecular weight of 400 Da (PEG400), 4 mL of ethanol (96% purity), and 12 mL of sterile water for injection. The dosing solution containing the 25% dispersion of compound (1-1) in PVP was used within 30 minutes after the addition of water. The dosing solutions containing the 25% dispersion of compound (1-1) in HPMCAS-MG and the 50% dispersion of compound (1-1) in HPMCAS-MG were used within 60 minutes of after the addition of water. A dosing volume of 4 mL/kg was used to give dose levels of compound (1-1) of 1 mg/kg for intravenous administration and 3 mg/kg for oral administration. The dosing scheme is given in Table 4.

Table 4. Dosing scheme for rat oral exposure study.

| Rat | Weight | Dose (mL) | Test Item | Route |
|---|---|---|---|---|
| 1 | 236.5 | 0.95 | Compound (1-1) | intravenous |
| 2 | 221 | 0.88 | Compound (1-1) | intravenous |
| 3 | 237.5 | 0.95 | Compound (1-1) | intravenous |
| | | | | |
| 4 | 255.5 | 1.02 | 25% dispersion of compound (1-1) in PVP | oral |
| 5 | 224.2 | 0.90 | 25% dispersion of compound (1-1) in PVP | oral |
| 6 | 219.2 | 0.88 | 25% dispersion of compound (1-1) in PVP | oral |

(continued)

| Rat | Weight | Dose (mL) | Test Item | Route |
|---|---|---|---|---|
| | | | | |
| 7 | 251.6 | 1.01 | 25% dispersion of compound (1-1) in HPMCAS-MG | oral |
| 8 | 240.4 | 0.96 | 25% dispersion of compound (1-1) in HPMCAS-MG | oral |
| 9 | 238 | 0.95 | 25% dispersion of compound (1-1) in HPMCAS-MG | oral |
| | | | | |
| 10 | 226.6 | 0.91 | 50% dispersion of compound (1-1) in HPMCAS-MG | oral |
| 11 | 228.4 | 0.91 | 50% dispersion of compound (1-1) in HPMCAS-MG | oral |
| 12 | 228.5 | 0.91 | 50% dispersion of compound (1-1) in HPMCAS-MG | oral |

Blood samples of approximately 50 μL were collected into Eppendorf tubes containing 5 μL of ethylenediaminetetraacetic acid (EDTA) solution at time points of 0.25, 0.5, 1, 2, 4, 8, 12, and 24 hours after dosing, with each sample collected within a window of 5 minutes from the prescribed time point. From each sample, 20 μL of plasma was obtained and stored at dry ice temperatures for analysis. Analysis of each sample for the concentration of compound (1-1) was performed using a validated liquid chromatography tandem mass spectrometry (LC-MS/MS) method with a lower limit of quantitation of 0.5 ng/mL.

Pharmacokinetic parameters were calculated with the Phoenix WinNonlin software package (version 6.2.1, Pharsight Corp., CA, USA) with standard noncompartmental methods. The elimination phase half-life ($t_{1/2}$) was calculated by least-squares regression analysis of the terminal linear part of the log concentration-time curve. The area under the plasma concentration-time curve (AUC) was determined by use of the linear trapezoidal rule up to the last measurable concentration and thereafter by extrapolation of the terminal elimination phase to infinity. The mean residence time (MRT), representing the average amount of time a compound remains in a compartment or system, was calculated by extrapolating the drug concentration profile to infinity. The maximum plasma concentration ($C_{max}$) and the time to $C_{max}$ ($t_{max}$) were derived directly from the plasma concentration data. The tentative oral bioavailability (F) was calculated by dividing the dose normalised AUC after oral administration by the dose normalised AUC after intravenous administration, i.e. F = (AUC(oral)/Dose(oral))/(AUC(intravenous) / Dose(intravenous))] and is reported as percentage (%).

The pharmacokinetic parameters are given in Table 5, and the plasma concentration versus time plots are shown in Figures 7 and 8.

Table 5. Pharmacokinetic parameters of compound (1-1) after oral and intravenous administrations. The values are an average from three animals.

| Compound | Parameter | 1 mg/kg intravenous | 3 mg/kg oral | F(%) |
|---|---|---|---|---|
| Compound (1-1) water:ethanol:PEG 400 (60:20:20) | AUC (min*ng/ml)<br>$C_{max}$ (ng/ml)<br>$T_{max}$ (hr)<br>$t_{1/2}$(hr) 8.5<br>Cl/F (ml/min/kg)<br>MRT (hr) | 74698<br>730<br>0.25<br>8.5<br>13.4<br>7.4 | | |
| 25% dispersion of compound (1-1) in PVP | AUC (min*ng/ml)<br>$C_{max}$ (ng/ml)<br>$T_{max}$ (hr)<br>$t_{1/2}$ (hr) 8.5<br>Cl/F (ml/min/kg)<br>MRT (hr) | | 39920<br>77.9<br>1<br>13.8<br>75.2<br>18.0 | 18 |

(continued)

| Compound | Parameter | 1 mg/kg intravenous | 3 mg/kg oral | F(%) |
|---|---|---|---|---|
| 25% dispersion of compound (1-1) in HPMCAS-MG | AUC (min*ng/ml)<br>$C_{max}$ (ng/ml)<br>$T_{max}$ (hr)<br>$t_{1/2}$(hr) 8.5<br>CI/F (ml/min/kg)<br>MRT (hr) | | 35306<br>48.3<br>0.5<br>11.0<br>85.0<br>17.1 | 16 |
| 50% dispersion of compound (1-1) in HPMCAS-MG | AUC (min*ng/ml)<br>$C_{max}$ (ng/ml)<br>$T_{max}$ (hr)<br>$t_{1/2}$(hr) 8.5<br>CI/F (ml/min/kg)<br>MRT (hr) | | 40238<br>67.0<br>2<br>9.5<br>74.6<br>12.8 | 18 |

Example 5. Preparation of spray dried dispersions.

Spray dried dispersions of compound (1-1) were prepared using five selected polymers: HPMCAS-MG (Shin Etsu Chemical Co., Ltd.), HPMCP-HP55 (Shin Etsu Chemical Co., Ltd.), PVP (ISP, a division of Ashland, Inc.), PVP-VA (BASF Corp.), and Eudragit L100-55 (Evonik Industries AG). All spray dried solutions were prepared at 25% and 50% by weight with each polymer. All solutions were prepared in acetone, with the exception of the PVP solutions, which were prepared in ethanol. For each solution, 1.0 g of solids (polymer and compound (1-1)) were prepared in 10 g of solvent. The solutions were spray dried using a Büchi B-290, PE-024 spray dryer with a 1.5 mm nozzle and a Büchi B-295, P-002 condenser. The spray dryer nozzle pressure was set to 80 psi, the target outlet temperature was set to 40 °C, the chiller temperature was set to -20 °C, the pump speed was set to 100%, and the aspirator setting was 100%. After spray drying, the solid dispersions were collected and dried overnight in a low temperature convection oven to remove residual solvents.

Example 6: Stability with humidity and temperature.

Table 6

<table>
<tr><th>Test</th><th>Procedure</th><th>Acceptance Criteria</th><th colspan="2">T=O (Initial)</th><th colspan="2">T-1 month (storage at 40°C/75%RH)</th><th colspan="2">T-2 month (storage at 40°C/ 75%RH)</th><th colspan="2">T= 3 month (storage at 40°C/75%RH)</th></tr>
<tr><td rowspan="2">Appearance</td><td rowspan="2">AM-0002</td><td rowspan="2">White to off-white powder</td><td colspan="2">Test Date/Ref: 06Aug2012/02-41-2</td><td colspan="2">Test Date/Ref: 24Sep2012/02-41-59</td><td colspan="2">Test Date/Ref: 240ct2012/02-37-106</td><td colspan="2">Test Date/Ref: 17Dec2012/02-37-119</td></tr>
<tr><td colspan="2">White Powder</td><td colspan="2">White Powder</td><td colspan="2">White Powder</td><td colspan="2">White Powder</td></tr>
<tr><td rowspan="2">Potency (HPLC)</td><td rowspan="2">AM-0028</td><td rowspan="2">45.0 • 55.0 wt%</td><td colspan="2">Test Date/Ref: 25Jul2012/02-37-21</td><td colspan="2">Test Date/Ref: 25Sep2012/02-4HI0</td><td colspan="2">Test Date/Ref: 24Oct2012/02-37-105</td><td colspan="2">Test Date/Ref: 29Nov2012/02-34-107</td></tr>
<tr><td colspan="2">50.0</td><td colspan="2">49.4</td><td colspan="2">49.8</td><td colspan="2">49.2</td></tr>
<tr><td rowspan="4">Individual Related Substances (HPLC)</td><td rowspan="4">AM-0029</td><td rowspan="4">Report results</td><td colspan="2">Test Date/Ref: 25Jul2012/02·34-49</td><td colspan="2">Test Date/Ref: 26Sep2012/02-41-64</td><td colspan="2">Test Date/Ref: 24Oct2012/02-37-105</td><td colspan="2">Test Date/Ref: 29Nov2012/02-34-107</td></tr>
<tr><td>RRT</td><td>% Area</td><td>RRT</td><td>% Area</td><td>RRT</td><td>% Area</td><td>RRT</td><td>% Area</td></tr>
<tr><td colspan="2" rowspan="2">No reportable related substances</td><td colspan="2" rowspan="2">No reportable related substances</td><td>0.68</td><td>0.06</td><td>0.68</td><td>0.07</td></tr>
<tr><td>0.77</td><td>0.06</td><td>0.77</td><td>0.09</td></tr>
<tr><td rowspan="2">Total Related Substances (HPLC)</td><td rowspan="2">AM·0029</td><td rowspan="2">Report results</td><td colspan="2">Test Date/Ref: 25Jul2012/02-34-49</td><td colspan="2">Test Date/Ref: 26Sep2012/02-41-64</td><td colspan="2">Test Date/Ref: 24Oct2012/02-37-105</td><td colspan="2">Test Date/Ref: 29Nov2012/02-34-107</td></tr>
<tr><td colspan="2">No reportable related substances</td><td colspan="2">No reportable related substances</td><td colspan="2">0.12%</td><td colspan="2">0.16%</td></tr>
<tr><td rowspan="2">Water Content (KF)</td><td rowspan="2">AM-0030 USP <921></td><td rowspan="2">Report results (wt%)</td><td colspan="2">Test Date/Ref: 02Aug2012/02-41-1</td><td colspan="2">Test Date/Ref: 27Sep2012/02-37-99</td><td colspan="2">Test Date/Ref: 25Oct2012102-37-110</td><td colspan="2">Test Date/Ref: 29Nov2012/0237-116</td></tr>
<tr><td colspan="2">1.52</td><td colspan="2">2.53</td><td colspan="2">2.70</td><td colspan="2">3.43</td></tr>
<tr><td rowspan="2">X-Ray Powder Diffraction (XRPD)</td><td rowspan="2">USP <941></td><td rowspan="2">Consistent with an amorphous form</td><td colspan="2">Test Date/Ref: 24Jul2012/02·24-131</td><td colspan="2">Test Date/Ref: 01Oct2012/02-41-73</td><td colspan="2">Test Date/Ref: 24Oct2012/02-37-107</td><td colspan="2">Test Date/Ref: 17Dec2012/02-37-120</td></tr>
<tr><td colspan="2">Consistent with an amorphous form See Figure 9</td><td colspan="2">Consistent with an amorphous form See Figure 10</td><td colspan="2">Consistent with an amorphous form See Figure 11</td><td colspan="2">Consistent with an amorphous form See Figure 12</td></tr>
</table>

(continued)

| Test | Procedure | Acceptance Criteria | T=O (Initial) | T-1 month (storage at 40°C/75%RH) | T-2 month (storage at 40°C/ 75%RH) | T= 3 month (storage at 40°C/75%RH) |
|---|---|---|---|---|---|---|
| Modulated Differential Scanning Calorimetry (mDSC) | USP <891> (n = 2 replicates) | Report individual and average glass transtion temperatures ($T_g$, °C) | Test Dale/Ref: 24Jul2012/02-24-130 | Test Date/Ref: 26Sep2012/02-37-98 | Test Date/Ref: 24Oct2012/02-37-108 | Test Date/Ref: 17Dec2012/02-37-121 |
| | | | Replicate 1 = 134.30°C, Replicate 2 = 134.23°C, Replicate 3 = 135.28°C, Average = 134.60°C | Replicate 1 = 134.65°C, Replicate 2 = 134.43°C, Average=134-54°C | Replicate 1 = 135.35°C, Replicate 2 = 134.93°C, Average = 135.14°C | Replicate 1 = 134.36°C. Replicate 2 = 137.16°C. Average= 135.76°C |

Spray dried dispersions of compound (1-1) in HPMCAS-MG were assessed for stability by exposure to moisture at elevated temperature. The glass transition temperature (Tg) as a function of relative humidity was determined at 75% relative humidity, 40 °C for 1, 2 and 3 months. The spray dried dispersion was stored in an LDPE bag inside a HDPE bottle to simulate bulk product packaging. The results are summarized in Table 6. At time zero, the Tg was 134 °C, at 1 month the Tg was 134 °C, at 2 months the Tg was 135 °C and at 3 months the Tg was 134 °C and only a single inflection point was observed for each measurement. X-ray diffraction patterns were also obtained for each sample. Figure 9 illustrates a powder X-ray diffraction profile of solid dispersions of compound (1-1) in HPMCAS-MG at time zero of a stability test. Figures 10, 11 and 12 illustrate powder X-ray diffraction profiles of solid dispersions of compound (1-1) in

HPMCAS-MG after 1 month, 2 months and 3 months, respectively, after exposure at 40 °C and 75 % relative humidity. The patterns did not show any diffraction lines associated with compound (1-1).

## Claims

1. A solid dispersion comprising an amorphous thienotriazolodiazepine compound which is (S)-2-[4-(4-chlorophenyl)-2,3,9-trimethyl-6H-thieno[3,2-f][1,2,-4]triazolo[4,3-a][1,4]diazepin-6-yl]-N-(4-hydroxyphenyl)acetamide, a pharmaceutically acceptable salt thereof or a hydrate thereof; and a pharmaceutically acceptable polymer, wherein the pharmaceutically acceptable polymer is hydroxypropylmethylcellulose acetate succinate and wherein the solid dispersion has a thienotriazolodiazepine compound to hydroxypropylmethylcellulose acetate succinate (HPMCAS), weight ratio of 1:3 to 1:1.

2. The solid dispersion according to claim 1, wherein the solid dispersion is obtained by spray drying.

3. The solid dispersion according to claim 1 or claim 2, wherein the solid dispersion exhibits a single glass transition temperature (Tg) inflection point ranging from about 130 °C to about 140 °C .

4. The solid dispersion according to claim 3, wherein the solid dispersion was exposed to a relative humidity of 75 % at 40 °C for at least one month.

5. The solid dispersion according to claim 4, wherein a concentration of the thienotriazolodiazepine compound after exposure to the relative humidity of 75 % at 40 °C for at least one month is at least 90 % of the concentration the amorphous thienotriazolodiazepine compound prior to such exposure.

6. The solid dispersion according to any of claims 1-5, wherein the solid dispersion exhibits an X-ray powder diffraction pattern substantially free of diffraction lines associated with crystalline thienotriazolodiazepine compound of claim 1.

7. The solid dispersion according to any of claims 1-6, wherein the solid dispersion provides an area under the curve (AUC) value that is at least 0.5 times that of a corresponding AUC value provided by a control composition administered intravenously, wherein the control composition comprises an equivalent quantity of a crystalline thienotriazolodiazepine compound of claim 1.

8. The solid dispersion according to any of claims 1-7, wherein the solid dispersion provides a concentration, of the amorphous thienotriazolodiazepine compound, in an aqueous in vitro test medium at pH between 5.0 to 7.0, of at least 5-fold greater than a concentration of a crystalline thienotriazolodiazepine compound of claim 1 without polymer, in a control in vitro test medium at pH between 5.0 to 7.0 test medium.

9. The solid dispersion according to any of claims 1-5, wherein a concentration of the amorphous thienotriazolodiazepine compound, from the solid dispersion, in an aqueous in vitro test medium having a pH of 1.0 to 2.0, is at least 50% higher than a concentration of a crystalline thienotriazolodiazepine compound of claim 1 without polymer in an in vitro test medium having a pH between 5.0 and 7.0.

10. The solid dispersion according to claim 1, wherein concentration of the amorphous thienotriazolodiazepine compound, is at least 50% higher compared to a concentration of thienotriazolodiazepine compound of claim 1, from a solid dispersion of thienotriazolodiazepine compound of the claim 1 and a pharmaceutically acceptable polymer selected from the group consisting of: hypromellose phthalate and ethyl acrylate-methyl methacrylate-trimethylammonioethyl methacrylate chloride copolymer, wherein each solid dispersion was placed in an aqueous in vitro test medium having a pH of 1.0 to 2.0.

**11.** A pharmaceutical formulation comprising a solid dispersion, according to any of claims 1-10, and one or more pharmaceutically acceptable excipients selected from the group consisting of:

lactose monohydrate; microcrystalline cellulose; croscarmellose sodium; colloidal silicon dioxide; magnesium stearate; and combinations thereof;
wherein said pharmaceutical formulation has a bulk density ranging from 0.55 g/cc to 0.60 g/cc.

**12.** A pharmaceutical capsule comprising the solid dispersion according to any of claims 1-10.

**13.** A pharmaceutical tablet comprising the solid dispersion according to any of claims 1-10.

**14.** A pharmaceutical formulation according to claim 11 comprising 10-15 wt. % of a solid dispersion; 45 -50 wt. % of lactose monohydrate; 35-40 wt. % of microcrystalline cellulose; 4-6 wt. % of croscarmellose sodium; 0.8-1.5 wt. % of colloidal silicon dioxide; and 0.8-1.5 wt. % of magnesium stearate.

**Patentansprüche**

**1.** Eine feste Dispersion, die eine amorphe Thienotriazoldiazepin-Verbindung, die (S)-2-[4-(4-Chlorphenyl)-2,3,9-trimethyl-6H-thieno[3,2-f][1,2,4]triazolo[4,3-a][1,4]-diazepin-6-yl]-N-(4-hydroxyphenyl)acetamid, ein pharmazeutisch annehmbares Salz davon oder ein Hydrat davon ist, und ein pharmazeutisch annehmbares Polymer umfasst, wobei das pharmazeutisch annehmbare Polymer Hydroxypropylmethylcellulose-Acetat-Succinat ist, und wobei die feste Dispersion ein Gewichtsverhältnis von Thienotriazoldiazepin-Verbindung zu Hydroxypropylmethylcellulose-Acetat-Succinat (HPMCAS) von 1:3 bis 1:1 besitzt.

**2.** Die feste Dispersion gemäß Anspruch 1, wobei die feste Dispersion durch Sprühtrocknen erhalten wird.

**3.** Die feste Dispersion gemäß Anspruch 1 oder Anspruch 2, wobei die feste Dispersion einen einzigen Glasübergangstemperatur(Tg)-Wendepunkt im Bereich von etwa 130°C bis etwa 140°C besitzt.

**4.** Die feste Dispersion gemäß Anspruch 3, wobei die feste Dispersion für wenigstens einen Monat einer relativen Feuchtigkeit von 75% bei 40°C ausgesetzt war.

**5.** Die feste Dispersion gemäß Anspruch 4, wobei eine Konzentration der Thienotriazoldiazepin-Verbindung, nachdem diese für wenigstens ein Monat der relativen Feuchtigkeit von 75% bei 40°C ausgesetzt war, wenigstens 90% der Konzentration der amorphen Thienotriazoldiazepin-Verbindung entspricht, bevor diese derart ausgesetzt war.

**6.** Die feste Dispersion gemäß einem der Ansprüche 1-5, wobei die feste Dispersion ein Röntgenpulverdiffraktogramm aufweist, das im Wesentlichen frei von Diffraktionslinien ist, welche zur kristallinen Thienotriazoldiazepin-Verbindung nach Anspruch 1 gehören.

**7.** Die feste Dispersion gemäß einem der Ansprüche 1-6, wobei die feste Dispersion einen Wert für die Fläche unter der Kurve (AUC) besitzt, der wenigstens das 0,5-fache eines entsprechenden AUC-Werts beträgt, den eine intravenös verabreichte Kontrollzusammensetzung besitzt, wobei die Kontrollzusammensetzung eine äquivalente Menge einer kristallinen Thienotriazoldiazepin-Verbindung nach Anspruch 1 umfasst.

**8.** Die feste Dispersion gemäß einem der Ansprüche 1-7, wobei die feste Dispersion eine Konzentration der amorphen Thienotriazoldiazepin-Verbindung in einem wässrigen In-vitro-Testmedium bei einem pH-Wert zwischen 5,0 und 7,0 bereitstellt, die wenigstens 5 Mal so groß ist wie eine Konzentration einer kristallinen Thienotriazoldiazepin-Verbindung nach Anspruch 1 ohne Polymer in einem Kontroll-In-vitro-Testmedium bei einem pH-Wert zwischen 5,0 und 7,0.

**9.** Die feste Dispersion gemäß einem der Ansprüche 1-5, wobei eine Konzentration der amorphen Thienotriazoldiazepin-Verbindung aus der festen Dispersion in einem wässrigen In-vitro-Testmedium mit einem pH-Wert von 1,0 bis 2,0 wenigstens 50% höher ist als eine Konzentration einer kristallinen Thienotriazoldiazepin-Verbindung nach Anspruch 1 ohne Polymer in einem In-vitro-Testmedium mit einem pH-Wert zwischen 5,0 und 7,0.

**10.** Die feste Dispersion gemäß Anspruch 1, wobei eine Konzentration der amorphen Thienotriazoldiazepin-Verbindung

wenigstens 50% höher ist, verglichen mit einer Konzentration der Thienotriazoldiazepin-Verbindung nach Anspruch 1 aus einer festen Dispersion von Thienotriazoldiazepin-Verbindung nach Anspruch 1 und einem pharmazeutisch annehmbaren Polymer, ausgewählt aus der Gruppe bestehend aus: Hypromellosephthalat und Ethylacrylat-Methylmethacrylat-Trimethylammoniumethylmethacrylatchlorid-Copolymer, wobei jede feste Dispersion in ein wässriges In-vitro-Testmedium mit einem pH-Wert von 1,0 bis 2,0 gegeben wurde.

**11.** Eine pharmazeutische Formulierung, umfassend eine feste Dispersion gemäß einem der Ansprüche 1-10 und einen oder mehrere pharmazeutisch annehmbare Arzneistoffträger, ausgewählt aus der Gruppe bestehend aus: Lactose-Monohydrat, mikrokristalliner Cellulose, Croscarmellose-Natrium, kolloidalem Siliziumdioxid, Magnesiumstearat und Kombinationen davon,
wobei die pharmazeutische Formulierung eine Schüttdichte im Bereich von 0,55 g/$cm^3$ bis 0,60 g/$cm^3$ besitzt.

**12.** Eine pharmazeutische Kapsel, die die feste Dispersion gemäß einem der Ansprüche 1-10 umfasst.

**13.** Eine pharmazeutische Tablette, die die feste Dispersion gemäß einem der Ansprüche 1-10 umfasst.

**14.** Eine pharmazeutische Formulierung gemäß Anspruch 11, die 10-15 Gew.-% einer festen Dispersion, 45-50 Gew.-% Lactose-Monohydrat, 35-40 Gew.-% mikrokristalline Cellulose, 4-6 Gew.-% Croscarmellose-Natrium, 0,8-1,5 Gew.-% kolloidales Siliziumdioxid und 0,8-1,5 Gew.-% Magnesiumstearat umfasst.

## Revendications

**1.** Dispersion solide comprenant un composé de thiénotriazolodiazépine amorphe qui est du (S)-2-[4-(4-chlorophényl)-2,3,9-triméthyl-6H-thiéno[3,2-f][1,2,-4]triazolo[4,3-a][1,4]diazépin-6-yl]-N-(4-hydroxyphényl)acétamide, un sel pharmaceutiquement acceptable de celui-ci ou un hydrate de celui-ci; et un polymère pharmaceutiquement acceptable, où le polymère pharmaceutiquement acceptable est du succinate-acétate d'hydroxypropylméthylcellulose et où la dispersion solide a un rapport pondéral de composé de thiénotriazolodiazépine à succinate-acétate d'hydroxypropylméthylcellulose (HPMCAS) de 1:3 à 1:1.

**2.** Dispersion solide selon la revendication 1, où la dispersion solide est obtenue par séchage par atomisation.

**3.** Dispersion solide selon la revendication 1 ou la revendication 2, où la dispersion solide présente un seul point d'inflexion de température de transition vitreuse (Tg) allant d'environ 130 °C à environ 140 °C.

**4.** Dispersion solide selon la revendication 3, où la dispersion solide a été exposée à une humidité relative de 75 % à 40 °C pendant un mois au moins.

**5.** Dispersion solide selon la revendication 4, dans laquelle une concentration du composé de thiénotriazolodiazépine après exposition à l'humidité relative de 75 % à 40 °C pendant un mois au moins, est d'au moins 90 % de la concentration du composé de thiénotriazolodiazépine amorphe préalablement à une telle exposition.

**6.** Dispersion solide selon l'une quelconque des revendications 1-5, où la dispersion solide présente un diagramme de diffraction des rayons X sur poudre sensiblement sans lignes de diffraction associées au composé de thiénotriazolodiazépine cristalline selon la revendication 1.

**7.** Dispersion solide selon l'une quelconque des revendications 1-6, où la dispersion solide donne une valeur d'aire sous la courbe (ASC) qui est d'au moins 0,5 fois celle d'une valeur ASC correspondante donnée par une composition témoin administrée par voie intraveineuse, où la composition témoin comprend une quantité équivalente d'un composé de thiénotriazolodiazépine cristalline selon la revendication 1.

**8.** Dispersion solide selon l'une quelconque des revendications 1-7, où la dispersion solide donne une concentration du composé de thiénotriazolodiazépine amorphe, dans un milieu de test in vitro aqueux à un pH d'entre 5,0 et 7,0, d'au moins 5 fois plus grande qu'une concentration d'un composé de thiénotriazolodiazépine cristalline selon la revendication 1 sans polymère, dans un milieu de test in vitro témoin à un pH d'entre 5,0 et 7,0 du milieu de test.

**9.** Dispersion solide selon l'une quelconque des revendications 1-5, dans laquelle une concentration du composé de thiénotriazolodiazépine amorphe, de la dispersion solide, dans un milieu de test in vitro aqueux ayant un pH d'entre

1,0 et 2,0, est au moins 50 % plus élevée qu'une concentration d'un composé de thiénotriazolodiazépine cristalline selon la revendication 1 sans polymère dans un milieu de test in vitro ayant un pH d'entre 5,0 et 7,0.

**10.** Dispersion solide selon la revendication 1, dans laquelle une concentration du composé de thiénotriazolodiazépine amorphe, est au moins 50 % plus élevée comparée à une concentration du composé de thiénotriazolodiazépine selon la revendication 1, d'une dispersion solide de composé de thiénotriazolodiazépine selon la revendication 1 et d'un polymère pharmaceutiquement acceptable sélectionné parmi le groupe consistant en: phtalate d'hypromellose et copolymère d'acrylate d'éthyle-méthacrylate de méthyle-chlorure de méthacrylate de triméthylammonioéthyle, où chaque dispersion solide a été placée dans un milieu de test in vitro aqueux ayant un pH de 1,0 à 2,0.

**11.** Formulation pharmaceutique comprenant une dispersion solide, selon l'une quelconque des revendications 1-10, et un ou plusieurs excipients pharmaceutiquement acceptables sélectionnés parmi le groupe consistant en: monohydrate de lactose; cellulose microcristalline; croscarmellose sodique; dioxyde de silicium colloïdal; stéarate de magnésium et des combinaisons de ceux-ci;
où ladite formulation pharmaceutique a une densité apparente allant de 0,55 g/cc à 0,60 g/cc.

**12.** Capsule pharmaceutique comprenant la dispersion solide selon l'une quelconque des revendications 1-10.

**13.** Comprimé pharmaceutique comprenant la dispersion solide selon l'une quelconque des revendications 1-10.

**14.** Formulation pharmaceutique selon la revendication 11, comprenant 10-15 % en poids d'une dispersion solide; 45-50 % en poids de monohydrate de lactose; 35-40 % en poids de cellulose microcristalline; 4-6 % en poids de croscarmellose sodique; 0,8-1,5 % en poids de dioxyde de silicium colloïdal et 0,8-1,5 % en poids de stéarate de magnésium.

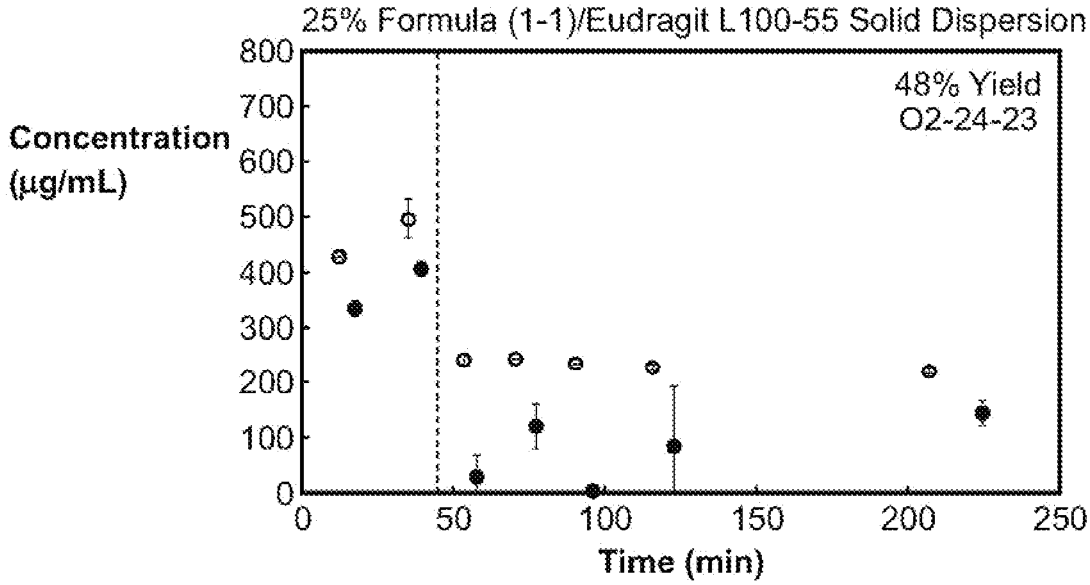
25% Formula (1-1)/Eudragit L100-55 Solid Dispersion
48% Yield
O2-24-23
Concentration (µg/mL)
800
700
600
500
400
300
200
100
0
0
50
100
150
200
250
Time (min)

FIG. 1A

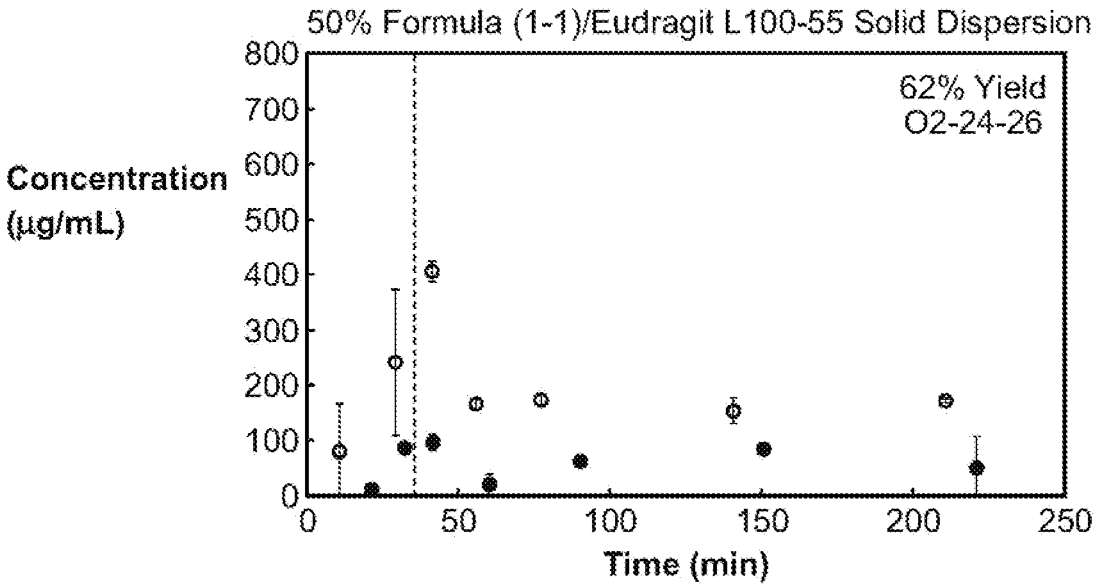
50% Formula (1-1)/Eudragit L100-55 Solid Dispersion
62% Yield
O2-24-26
Concentration (µg/mL)
800
700
600
500
400
300
200
100
0
0
50
100
150
200
250
Time (min)

FIG. 1B

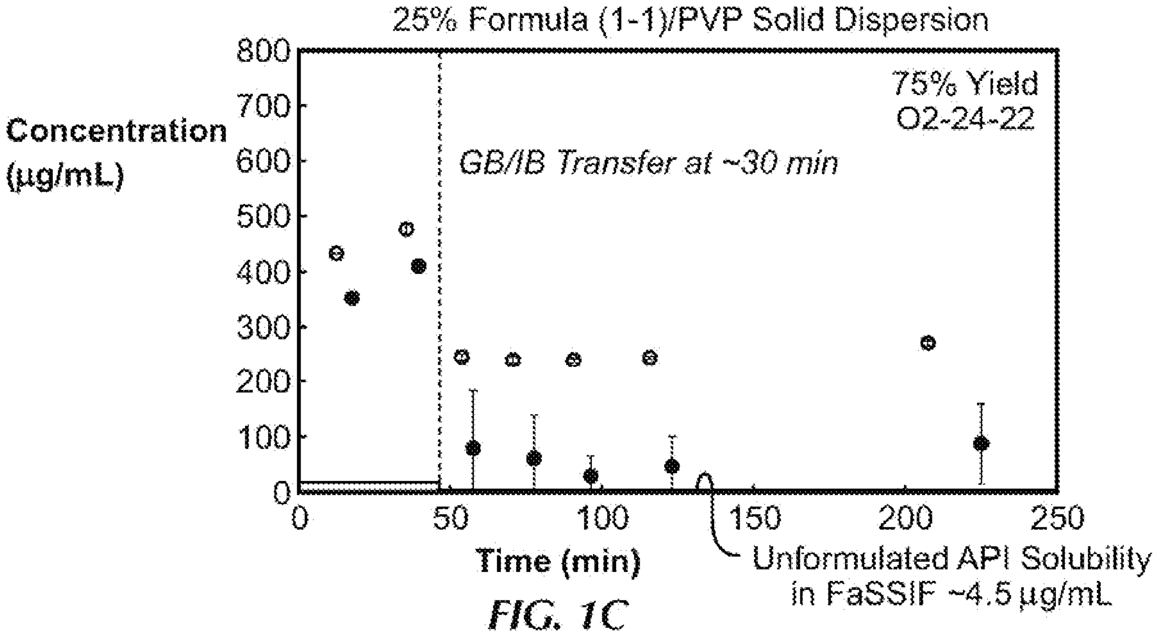
25% Formula (1-1)/PVP Solid Dispersion
Concentration (μg/mL)
800
700
600
500
400
300
200
100
0
75% Yield
O2-24-22
GB/IB Transfer at ~30 min
0
50
100
150
200
250
Time (min)
Unformulated API Solubility in FaSSIF ~4.5 μg/mL

*FIG. 1C*

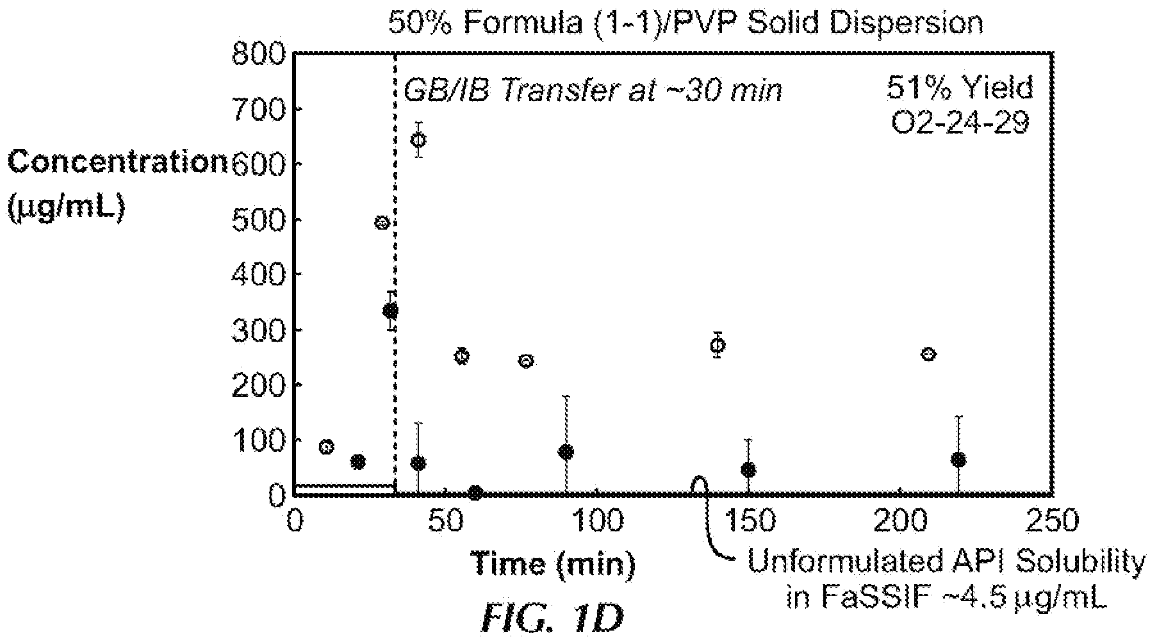
50% Formula (1-1)/PVP Solid Dispersion
Concentration (μg/mL)
800
700
600
500
400
300
200
100
0
GB/IB Transfer at ~30 min
51% Yield
O2-24-29
0
50
100
150
200
250
Time (min)
Unformulated API Solubility in FaSSIF ~4.5 μg/mL

*FIG. 1D*

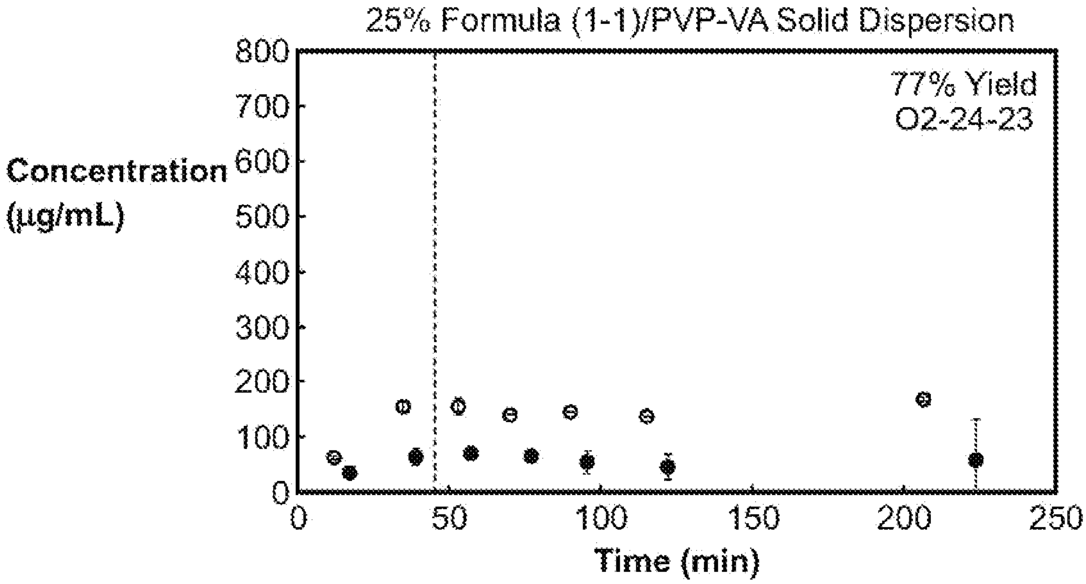
25% Formula (1-1)/PVP-VA Solid Dispersion
77% Yield
O2-24-23
Concentration (µg/mL)
800
700
600
500
400
300
200
100
0
0
50
100
150
200
250
Time (min)

*FIG. 1E*

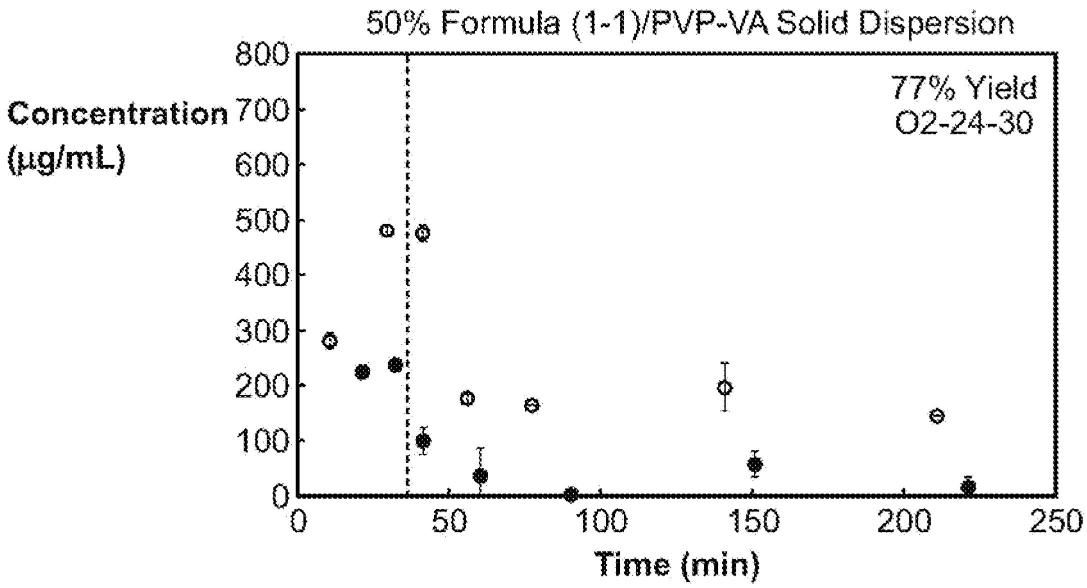
50% Formula (1-1)/PVP-VA Solid Dispersion
77% Yield
O2-24-30
Concentration (µg/mL)
800
700
600
500
400
300
200
100
0
0
50
100
150
200
250
Time (min)

*FIG. 1F*

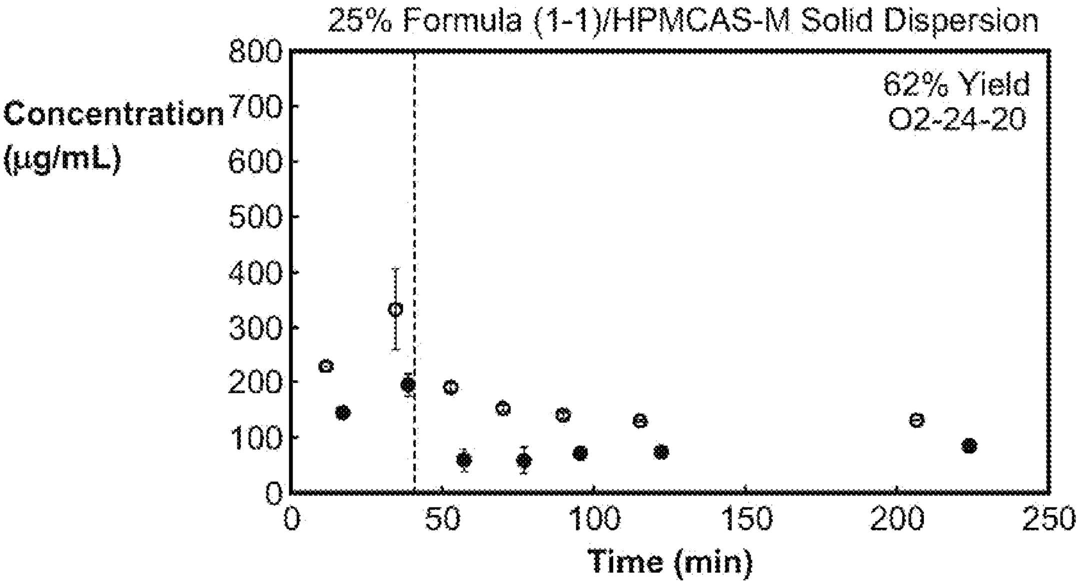
25% Formula (1-1)/HPMCAS-M Solid Dispersion
Concentration (µg/mL)
800
700
600
500
400
300
200
100
0
62% Yield
O2-24-20
0
50
100
150
200
250
Time (min)

*FIG. 1G*

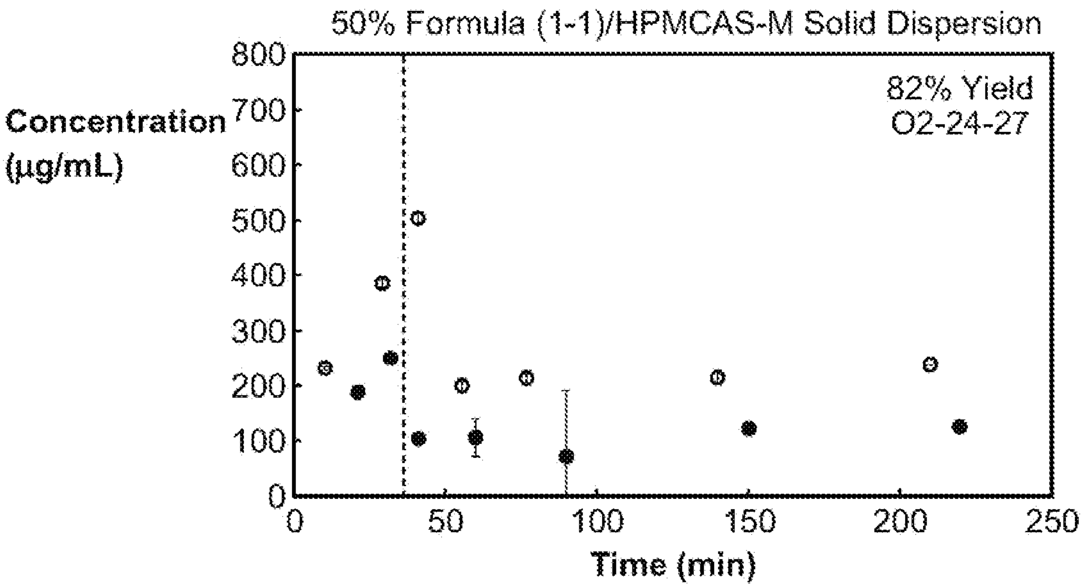
50% Formula (1-1)/HPMCAS-M Solid Dispersion
Concentration (µg/mL)
800
700
600
500
400
300
200
100
0
82% Yield
O2-24-27
0
50
100
150
200
250
Time (min)

*FIG. 1H*

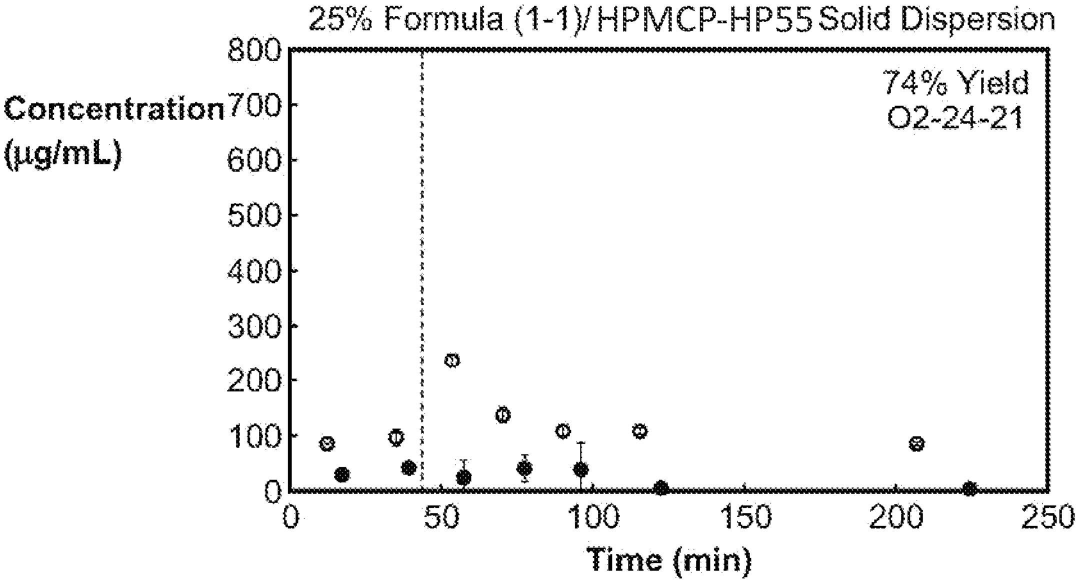
25% Formula (1-1)/HPMCP-HP55 Solid Dispersion
74% Yield
O2-24-21
Concentration (µg/mL)
0
100
200
300
400
500
600
700
800
Time (min)
0
50
100
150
200
250

*FIG. 1I*

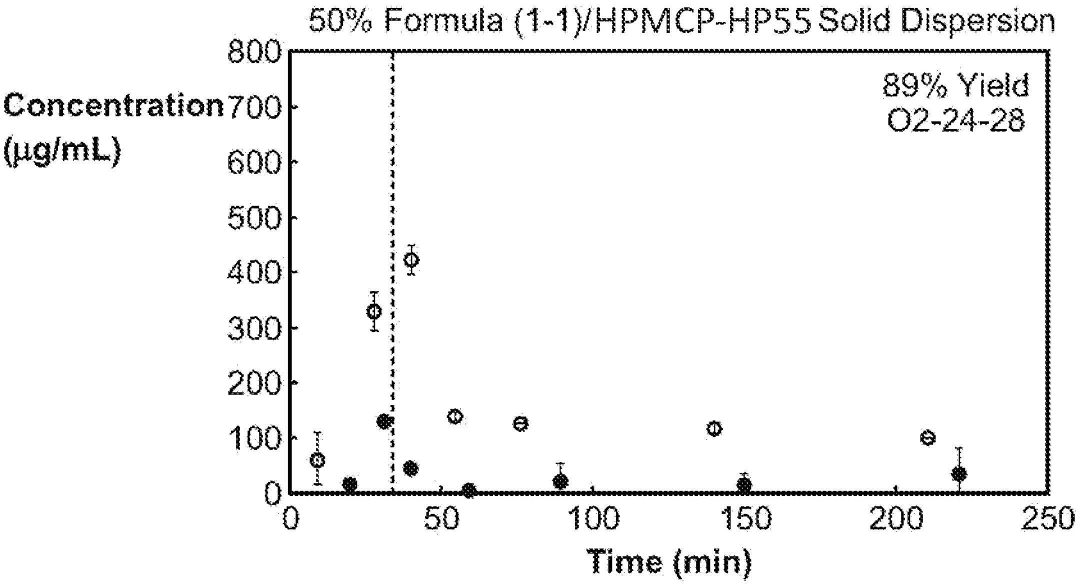
50% Formula (1-1)/HPMCP-HP55 Solid Dispersion
89% Yield
O2-24-28
Concentration (µg/mL)
0
100
200
300
400
500
600
700
800
Time (min)
0
50
100
150
200
250

*FIG. 1J*

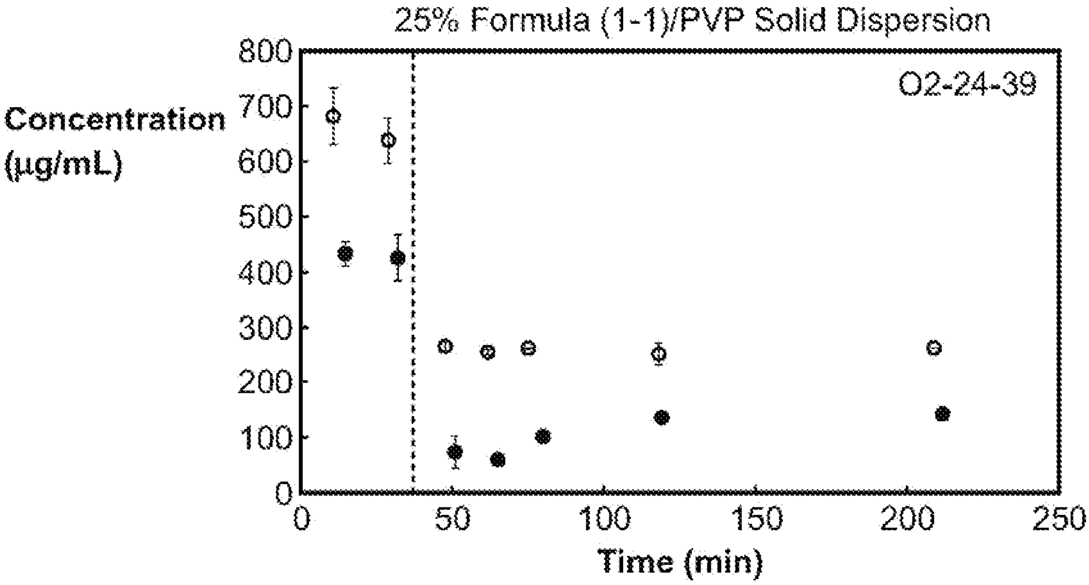
25% Formula (1-1)/PVP Solid Dispersion
O2-24-39
Concentration (µg/mL)
800
700
600
500
400
300
200
100
0
0
50
100
150
200
250
Time (min)

*FIG. 2A*

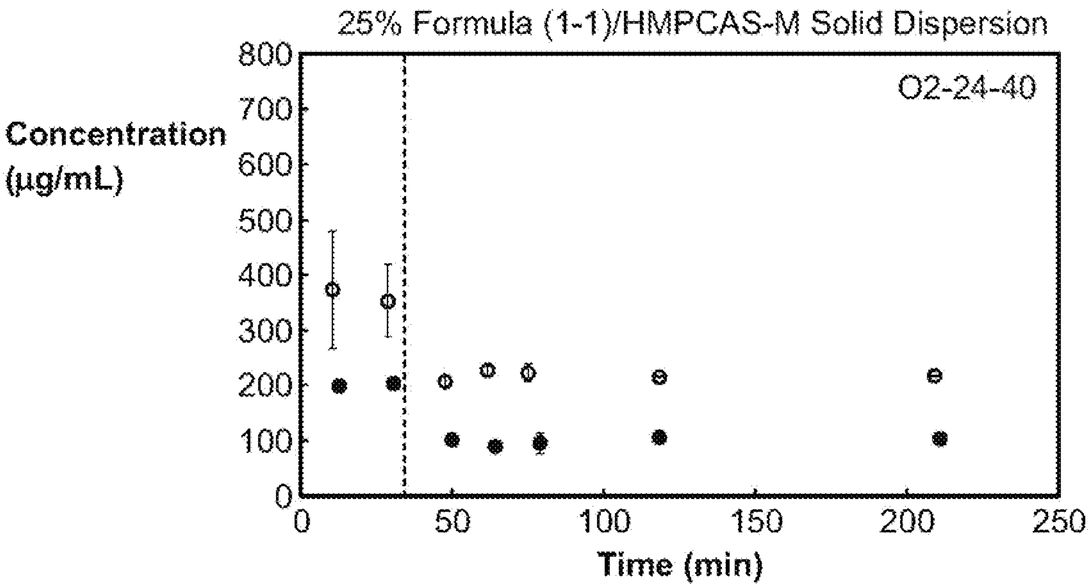
25% Formula (1-1)/HMPCAS-M Solid Dispersion
O2-24-40
Concentration (µg/mL)
800
700
600
500
400
300
200
100
0
0
50
100
150
200
250
Time (min)

*FIG. 2B*

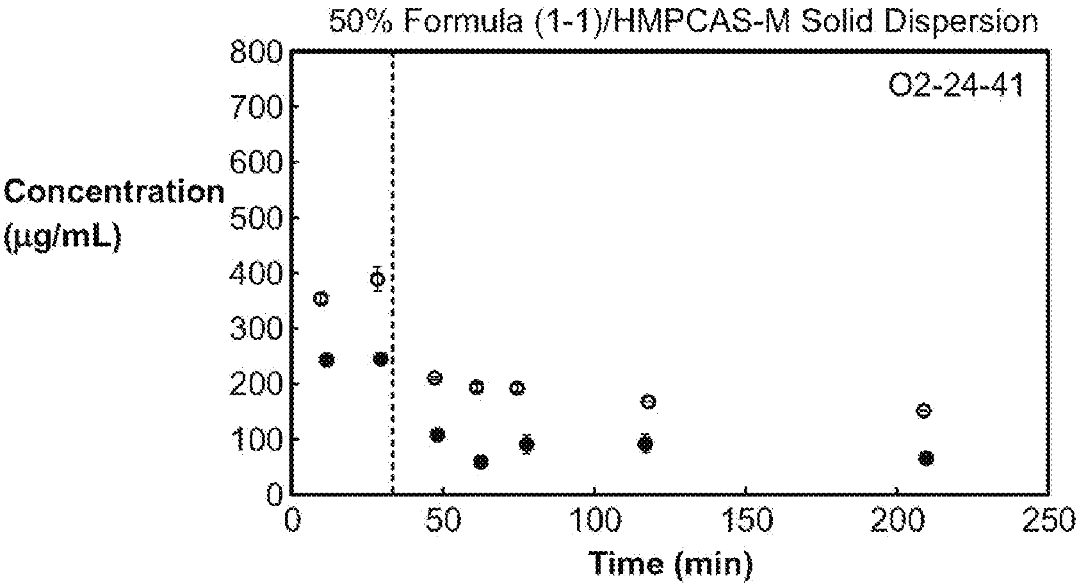
50% Formula (1-1)/HMPCAS-M Solid Dispersion
O2-24-41
Concentration (µg/mL)
800
700
600
500
400
300
200
100
0
0
50
100
150
200
250
Time (min)

*FIG. 2C*

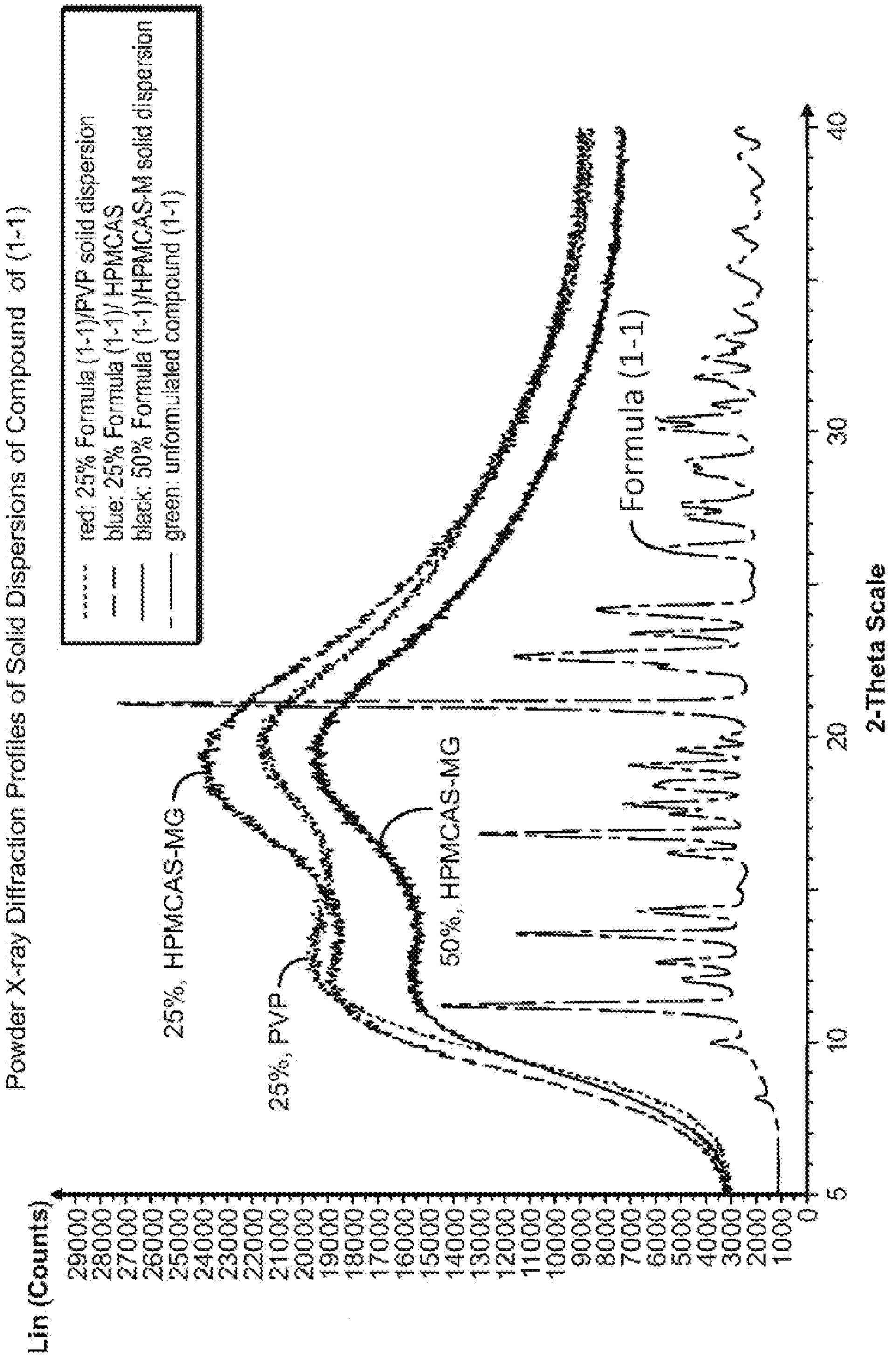
Powder X-ray Diffraction Profiles of Solid Dispersions of Compound of (1-1)
red: 25% Formula (1-1)/PVP solid dispersion
blue: 25% Formula (1-1)/ HPMCAS
black: 50% Formula (1-1)/HPMCAS-M solid dispersion
green: unformulated compound (1-1)
Lin (Counts)
29000
28000
27000
26000
25000
24000
23000
22000
21000
20000
19000
18000
17000
16000
15000
14000
13000
12000
11000
10000
9000
8000
7000
6000
5000
4000
3000
2000
1000
0
25%, HPMCAS-MG
25%, PVP
50%, HPMCAS-MG
Formula (1-1)
5
10
20
30
40
2-Theta Scale

*FIG. 3*

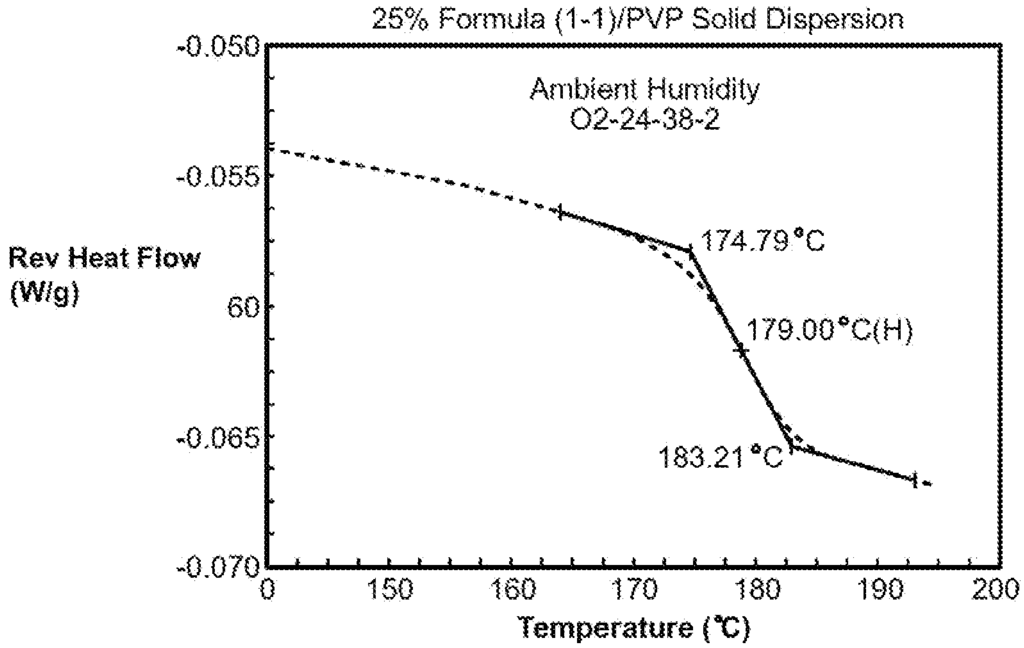
25% Formula (1-1)/PVP Solid Dispersion
Ambient Humidity
O2-24-38-2
174.79°C
179.00°C(H)
183.21°C
Rev Heat Flow
(W/g)
-0.050
-0.055
60
-0.065
-0.070
0
150
160
170
180
190
200
Temperature (°C)

FIG. 4A

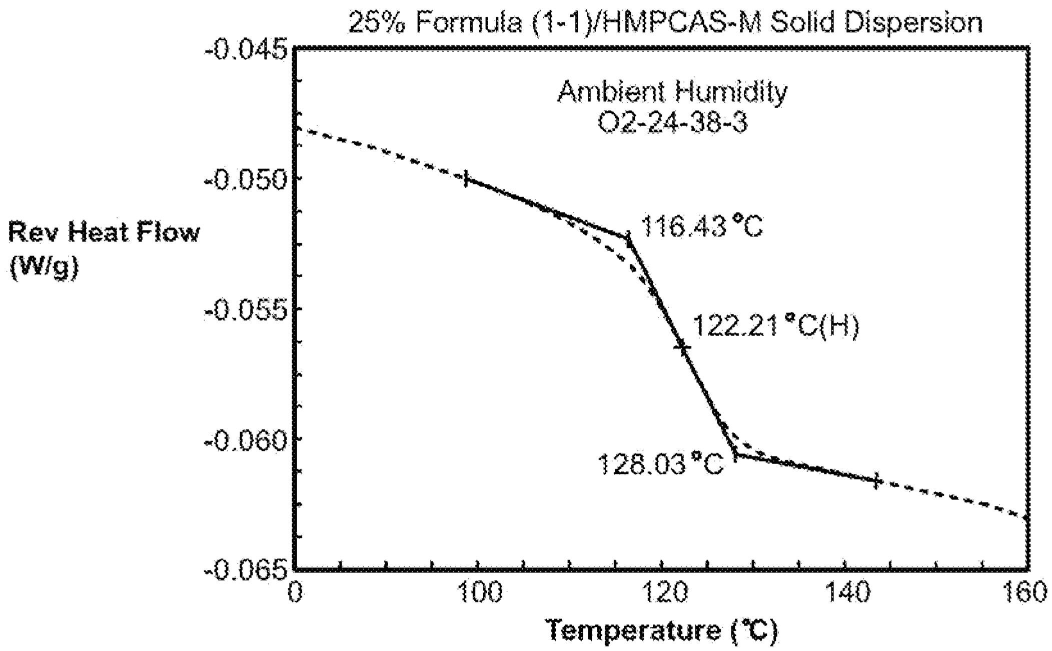
25% Formula (1-1)/HMPCAS-M Solid Dispersion
Ambient Humidity
O2-24-38-3
116.43°C
122.21°C(H)
128.03°C
Rev Heat Flow
(W/g)
-0.045
-0.050
-0.055
-0.060
-0.065
0
100
120
140
160
Temperature (°C)

FIG. 4B

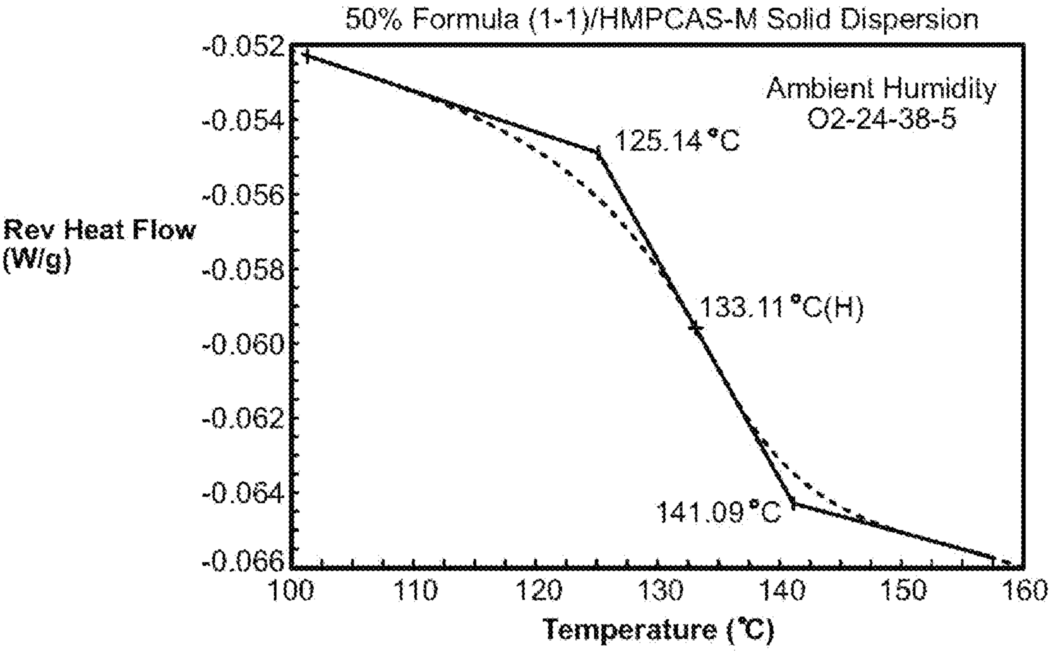
50% Formula (1-1)/HMPCAS-M Solid Dispersion
Ambient Humidity
O2-24-38-5
125.14 °C
133.11 °C(H)
141.09 °C
Rev Heat Flow (W/g)
-0.052
-0.054
-0.056
-0.058
-0.060
-0.062
-0.064
-0.066
100
110
120
130
140
150
160
Temperature (°C)

*FIG. 4C*

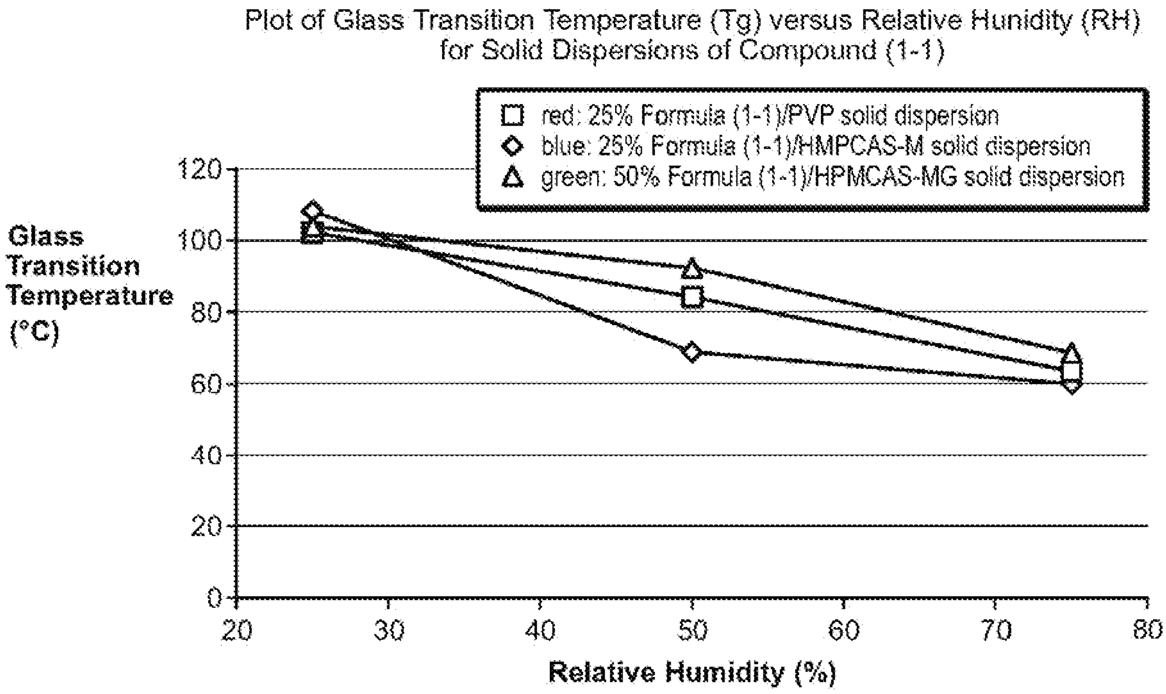
Plot of Glass Transition Temperature (Tg) versus Relative Hunidity (RH)
for Solid Dispersions of Compound (1-1)
red: 25% Formula (1-1)/PVP solid dispersion
blue: 25% Formula (1-1)/HMPCAS-M solid dispersion
green: 50% Formula (1-1)/HPMCAS-MG solid dispersion
Glass Transition Temperature (°C)
120
100
80
60
40
20
0
20
30
40
50
60
70
80
Relative Humidity (%)

*FIG. 5*

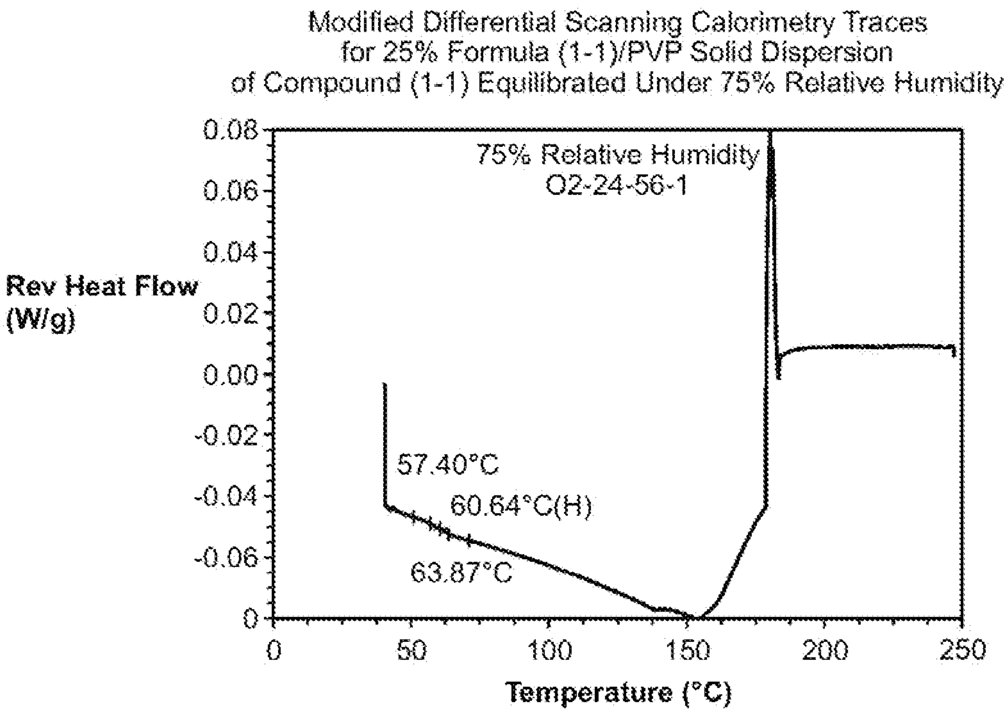
Modified Differential Scanning Calorimetry Traces
for 25% Formula (1-1)/PVP Solid Dispersion
of Compound (1-1) Equilibrated Under 75% Relative Humidity
75% Relative Humidity
O2-24-56-1
Rev Heat Flow (W/g)
0.08
0.06
0.04
0.02
0.00
-0.02
-0.04
-0.06
0
57.40°C
60.64°C(H)
63.87°C
0
50
100
150
200
250
Temperature (°C)

*FIG. 6*

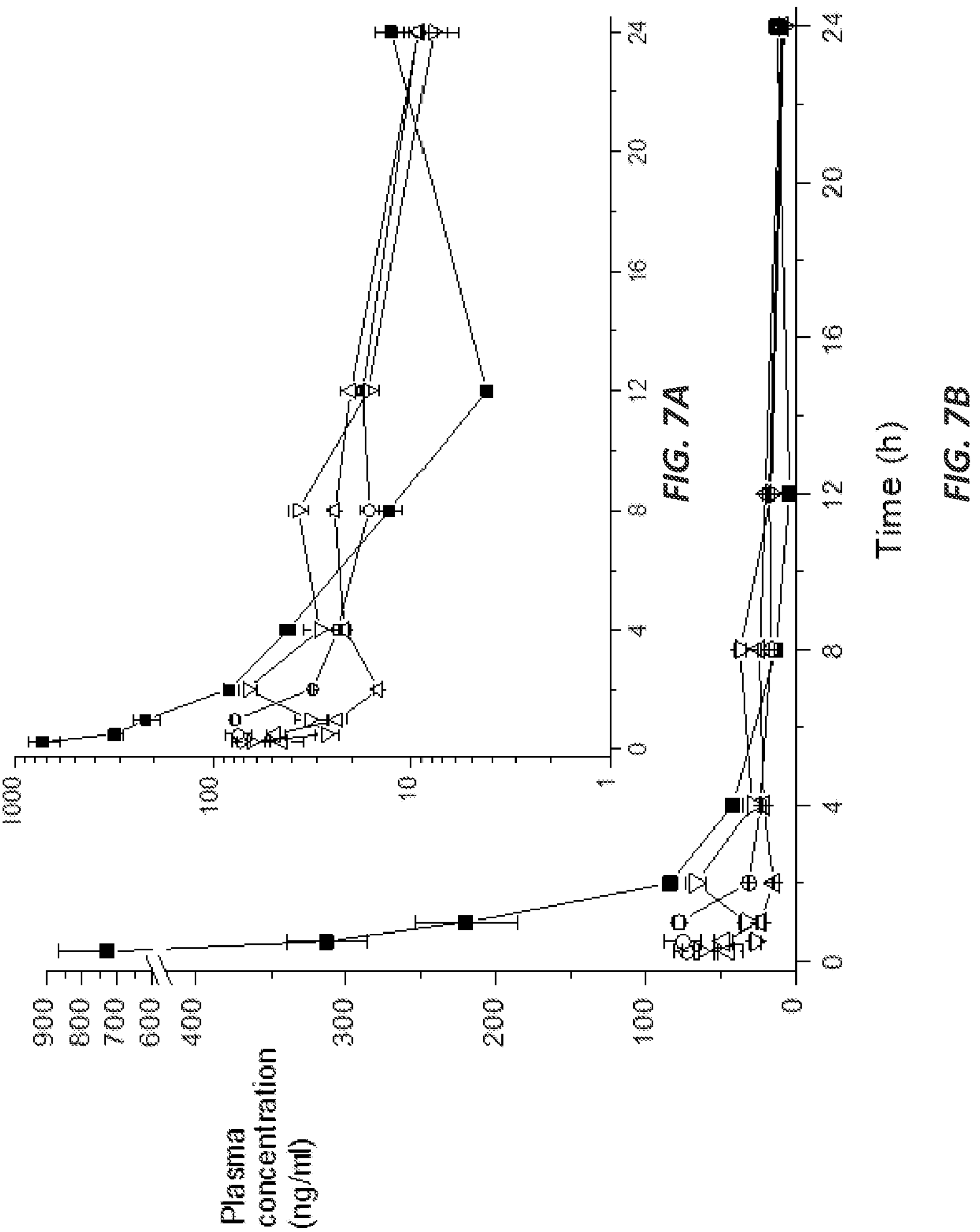
Plasma concentration (ng/ml)
Time (h)
0
4
8
12
16
20
24
1
10
100
1000
0
100
200
300
400
600
700
800
900

FIG. 7A

FIG. 7B

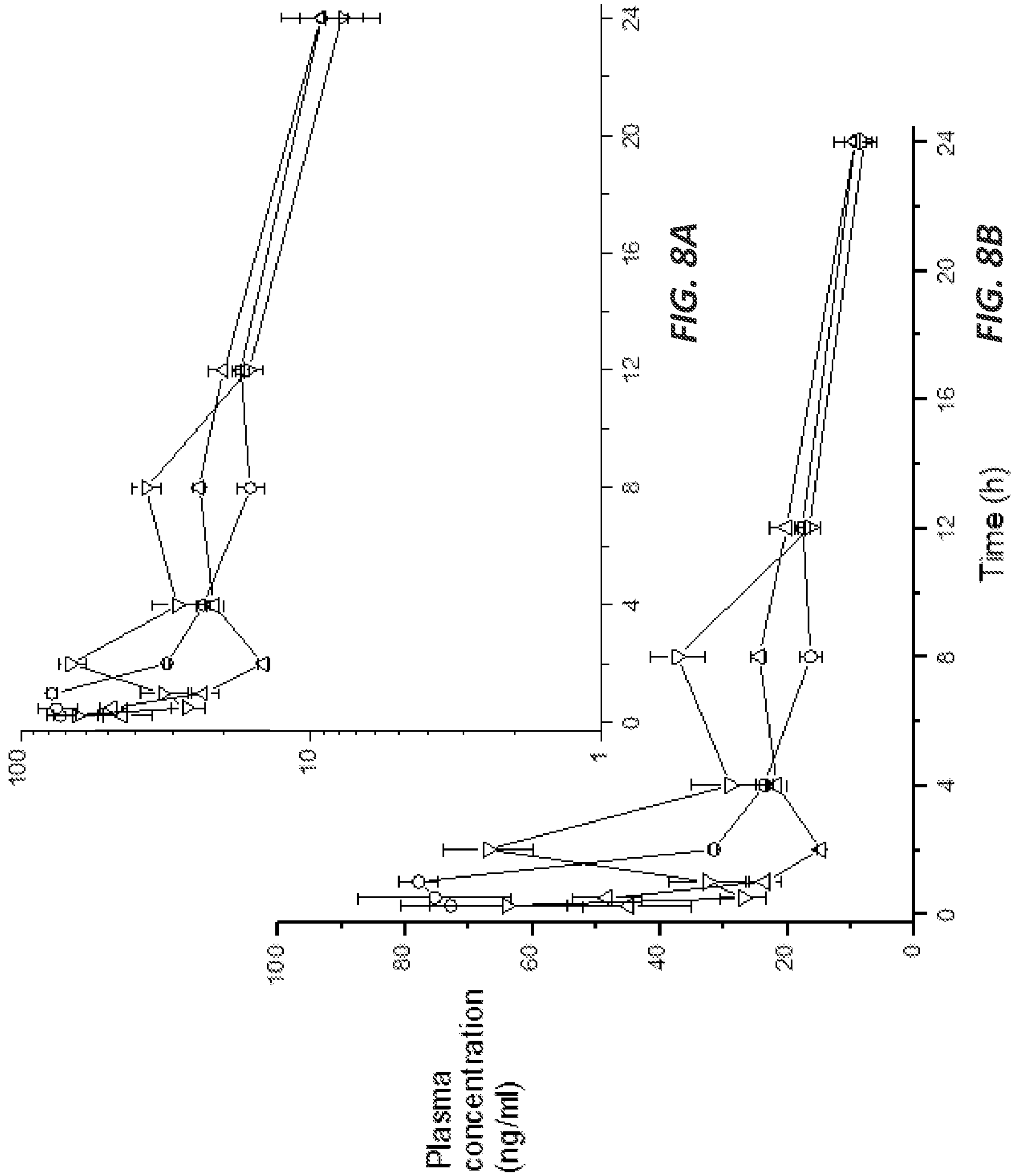

100
10
1
0
4
8
12
16
20
24
Plasma concentration (ng/ml)
100
80
60
40
20
0
Time (h)


FIG. 8A

FIG. 8B

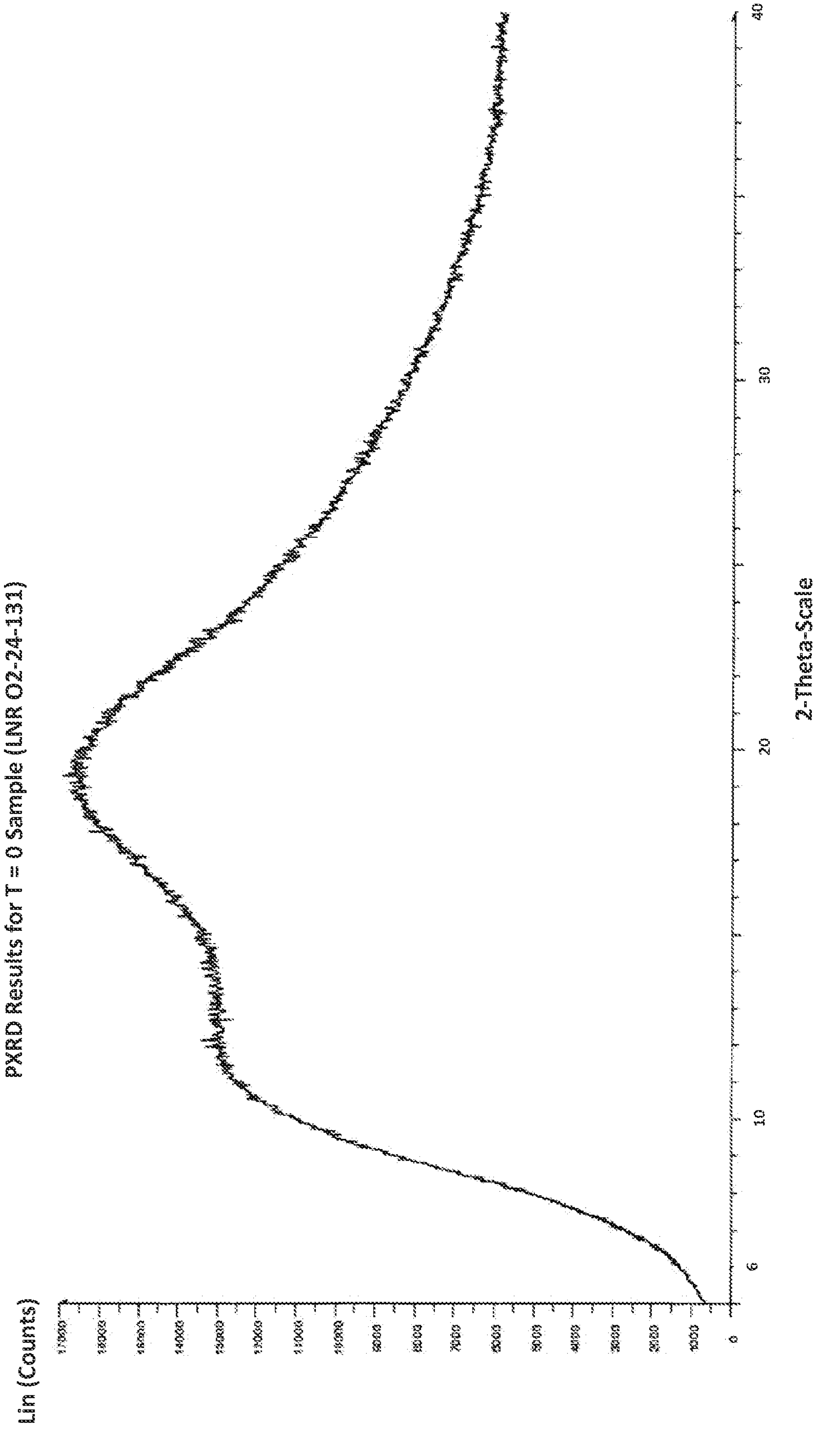
PXRD Results for T = 0 Sample (LNR O2-24-131)
Lin (Counts)
2-Theta-Scale
0
6
10
20
30
40

FIG. 9

PXRD Results for 1 month Stability Sample (LNR O2-41-73)

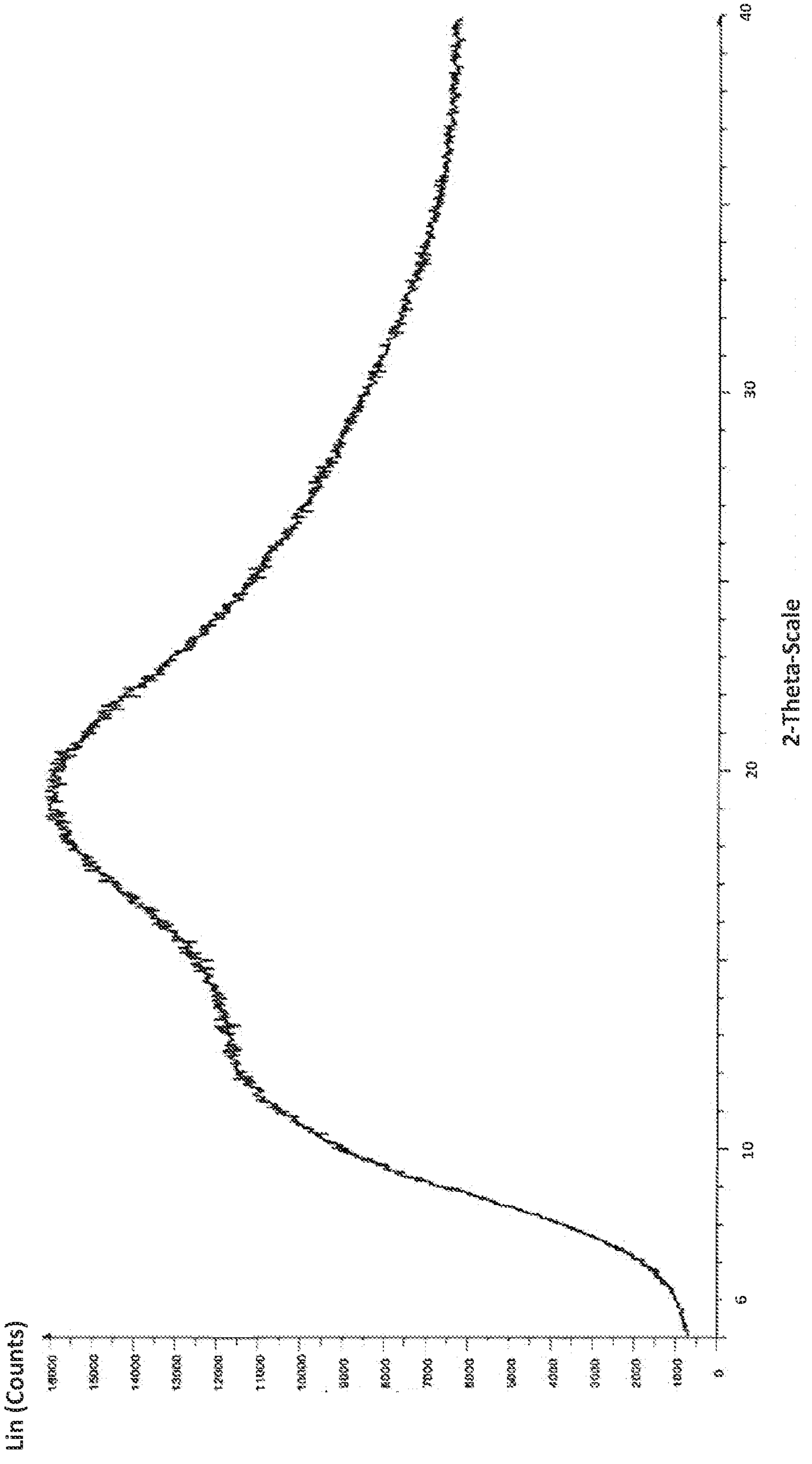


*FIG. 10*

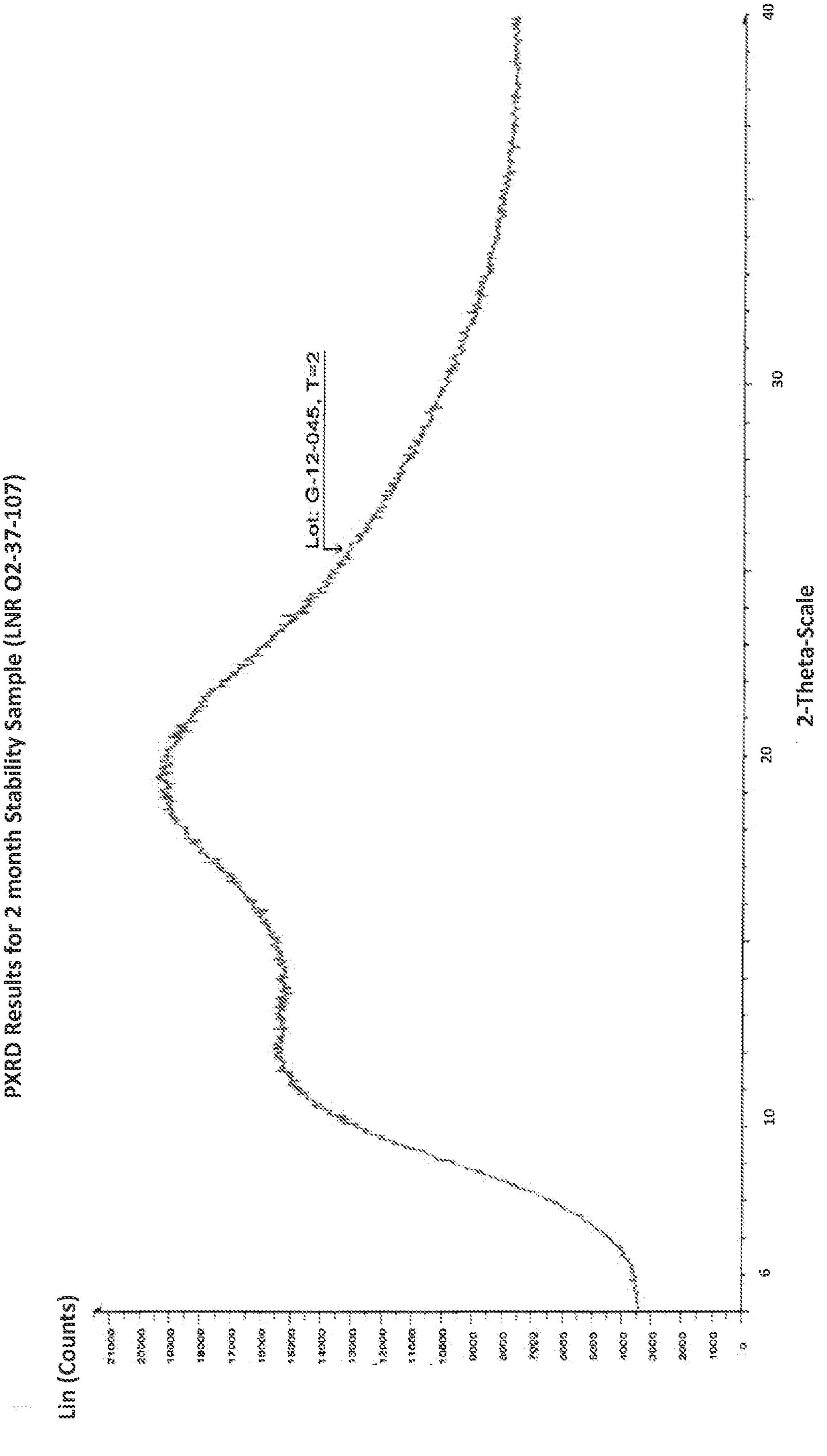
PXRD Results for 2 month Stability Sample (LNR O2-37-107)
Lin (Counts)
Lot: G-12-045, T=2
2-Theta-Scale
10
20
30
40

*FIG. 11*

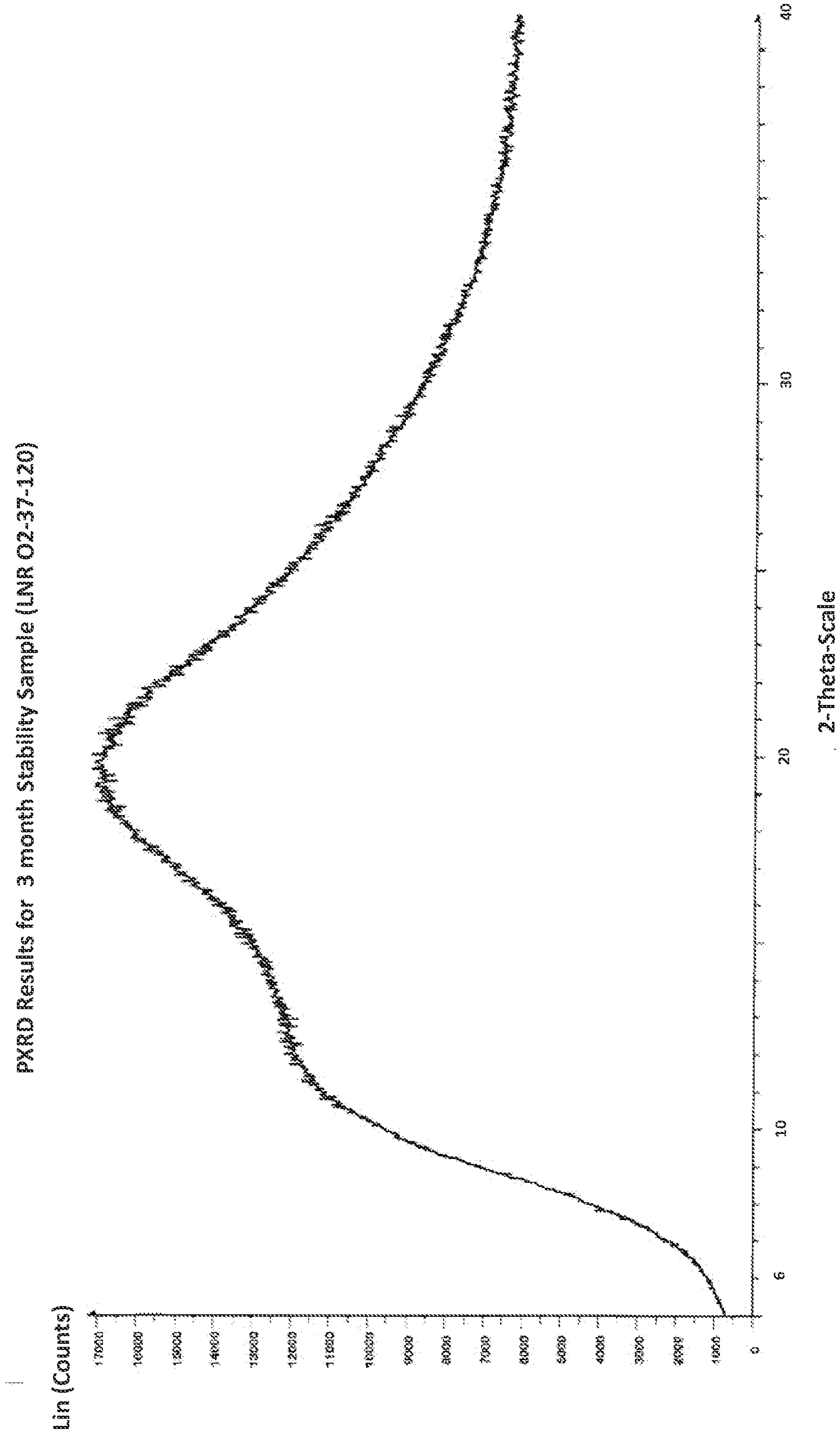
PXRD Results for 3 month Stability Sample (LNR O2-37-120)
Lin (Counts)
17000
16000
15000
14000
13000
12000
11000
10000
9000
8000
7000
6000
5000
4000
3000
2000
1000
0
6
10
20
30
40
2-Theta-Scale

*FIG. 12*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20100286127 A1
- EP 1297836 A1
- US 20090012064 A1
- US 20100286127 A


### Non-patent literature cited in the description


- **DENIS, G.V.** Bromodomain coactivators in cancer, obesity, type 2 diabetes, and inflammation. *Discov Med,* 2010, vol. 10, 489-499
- **CARREIRA, E. M. ; KVAERNO, L.** Classics in Stereoselective Synthesis. Wiley-VCH, 2009
- **CHIOU WL ; RIEGELMAN S.** Pharmaceutical applications of solid dispersion systems. *J. Pharm. Sci.,* 1971, vol. 60, 1281-1302
- **SERAJUDDIN ATM.** Solid dispersion of poorly water-soluble drugs: early promises, subsequent problems, and recentbreakthroughs. *J. Pharm. Sci.,* 1999, vol. 88, 1058-1066
- **LEUNER C ; DRESSMAN J.** Improving drug solubility for oral delivery using solid dispersions. *Eur. J. Pharm. Biopharm.,* 2000, vol. 50, 47-60
- **VASCONCELOS T ; SARMENTO B ; COSTA P.** Solid dispersions as strategy to improve oral bioavailability of poor water soluble drugs. *Drug Discovery Today,* 2007, vol. 12, 1068-1075